# EUROPEAN PATENT APPLICATION

(11) **EP 3 906 951 A1**
(43) Date of publication of application: **10.11.2021**
(21) Application number: 21174075.8
(22) Date of filing: 12.12.2014
(51) Int. Cl.: A61M 5/142, A61M 39/04, A61M 5/145, A61M 5/28, A61M 39/02, A61M 5/31

(54) **ON-BODY INJECTOR**

(30) Priority: 19.12.2013 US 201314134749
(62) Divisional of application: 19193719.2
(71) Applicant: Medtronic MiniMed, Inc., Northridge, CA 91325-1219 (US)
(72) Inventor: CHONG, Colin A., Glendale, CA 91207 (US); SCHULHAUSER, Randal C., Phoenix, 85048 (US); STEVENSON, Tyler, Westminster, CO 80020 (US); BIKOVSKY, Rafael, Oak Park, 91377 (US)
(74) Representative: Madgwick, Paul Roland

(57) **Abstract**

An on-body injector (192) for use with a patient with an injection device having an injection port in fluid communication with a delivery tube (150), the injection port (140) lying on an injection axis (144), the on-body injector (192) comprising: a bolus reservoir (1100); a bolus injection needle (1040) in fluid communication with the bolus reservoir (1100), the bolus injection needle (1040) having a bolus injection needle tip (1042) aligned with the injection port, the bolus injection needle being slideably biased away from the injection port (140) to define a gap (1120) between the bolus injection needle tip (1042) and the injection port (140); and a button (1030) operably connected to the bolus injection needle (1040) to slide the bolus injection needle (1040) along the injection axis (144); wherein the button (1030) is operable to advance the bolus injection needle tip (1042) to close the gap (1120) and advance the bolus injection needle tip into the injection port to form an injection flow path from the bolus reservoir (1100), through the bolus injection needle (1040), through the delivery tube (150), and into the patient; wherein the button (1030) is further operable to advance a plunger (1110) through the bolus reservoir (1100) to deliver a predetermined bolus volume to the patient through the injection flow path; and a pressurized reservoir (1070) in fluid communication with the bolus reservoir.

## Description

### CROSS REFERENCES TO RELATED APPLICATIONS

This application is a continuation-in-part of and claims the benefit of U.S. Patent Application Serial No. 14/052,929, filed on October 14, 2013, entitled THERAPEUTIC AGENT INJECTION DEVICE, which is incorporated by reference in its entirety herein.

### TECHNICAL FIELD

The technical field of this disclosure is personal medical systems, particularly, on-body injectors and methods of use.

### BACKGROUND OF THE INVENTION

Certain medical conditions or diseases require that patients intermittently inject a drug or therapeutic agent subcutaneously to maintain the medical condition or disease under control. Multiple daily injections (MDIs) may be required. One such medical condition is diabetes, for which insulin is injected to regulate blood glucose. An estimated twenty-six million people in the United States, or about 8% of the population, have diabetes. This percentage is expected to increase in the near-term as the population ages.

Certain patients are unlikely or unable to follow the drug regimen required to maintain their medical condition under control. Some patients are squeamish about injecting the drug themselves and others suffer adverse effects from repeated injections, such as bruising at the injection site. To accommodate such patients, injection ports have been developed which only require that the patient puncture their skin every few days to install an injection port, rather than injecting with a needle into their skin numerous times a day. Injection ports employ a cannula inserted subcutaneously, and the patient injects the drug into the injection port adhering to their skin rather than directly into their cutaneous tissue.

Unfortunately, injection ports still require that the patient administer the therapeutic agent repeatedly throughout the day. Injection ports with dedicated reservoirs allowing bolus injection have been developed, but maintain a continuous flow path and run the risk of inadvertent bolus injection. Although these problems can be remedied with a dedicated electronic insulin pump, many patients are unwilling or unable to use a dedicated insulin pump due to the expense and complication. Such systems present other obstacles to the patient, such as the inability to choose different therapy options based on specific daily needs or activity.

It would be desirable to have an on-body injectors and methods of use that would overcome the above disadvantages.

### SUMMARY OF THE INVENTION

One aspect of the invention provides an on-body injector for use with a patient with an injection device having an injection port in fluid communication with a delivery tube and the injection port lying on an injection axis. The on-body injector includes a bolus reservoir; a bolus injection needle in fluid communication with the bolus reservoir, the bolus injection needle having a bolus injection needle tip aligned with the injection port, the bolus injection needle being slideably biased away from the injection port to define a gap between the bolus injection needle tip and the injection port; and a button operably connected to the bolus injection needle to slide the bolus injection needle along the injection axis. The button is operable to advance the bolus injection needle tip to close the gap and advance the bolus injection needle tip into the injection port to form an injection flow path from the bolus reservoir, through the bolus injection needle, through the delivery tube, and into the patient. The button is further operable to advance a plunger through the bolus reservoir to deliver a predetermined bolus volume to the patient through the injection flow path.

Another aspect of the invention provides an on-body injector for use with a patient with an injection device having an introducer port in fluid communication with a delivery tube. The on-body injector includes a pressurized reservoir; a flow restrictor disposed between the pressurized reservoir and the delivery tube, the flow restrictor being tubing having a length and interior diameter selected to provide a desired pressure drop; and a fill port in fluid communication with the pressurized reservoir.

Another aspect of the invention provides a method of use for an on-body injector with an injection device for delivering a predetermined bolus volume to a patient, the method including deploying the injection device in the patient, the injection device having a delivery tube placed in the patient and an injection port in fluid communication with the delivery tube; securing the on-body injector to the injection device, the on-body injector having a bolus injection needle aligned with and spaced apart from the injection port; depressing a button on the on-body injector to advance the bolus injection needle into the injection port; and further depressing the button to deliver the predetermined bolus volume from the on-body injector through the bolus injection needle, through the delivery tube, and into the patient.

The foregoing and other features and advantages of the invention will become further apparent from the following detailed description of the presently preferred embodiments, read in conjunction with the accompanying drawings. The detailed description and drawings are merely illustrative of the invention, rather than limiting the scope of the invention being defined by the appended claims and equivalents thereof.

### BRIEF DESCRIPTION OF THE DRAWINGS

**FIGS. 1-4** are perspective, section, perspective, and exploded perspective views, respectively, of one embodiment of an injection device made in accordance with the invention.
**FIGS. 5A-5C** are perspective views of one embodiment of an injection device made in accordance with the invention.
**FIG. 6** is a section perspective view of one embodiment of an injection device made in accordance with the invention.
**FIGS. 7-11** are perspective, perspective, section, section, and perspective section views, respectively, of one embodiment of an injection device made in accordance with the invention.
**FIGS. 12A-12D** are side and section views of needleless pen injectors for use with an injection device made in accordance with the invention.
**FIGS. 13A-13F** are section views of pop-up indicator ports for use with an injection device made in accordance with the invention.
**FIGS. 14A** **&** **14B** are perspective views of one embodiment of an injection device made in accordance with the invention.
**FIGS. 15-20** are front perspective, top side, left side, bottom side, bottom perspective, and detail views, respectively, of one embodiment of a body for an injection device made in accordance with the invention.
**FIG. 21** is a perspective view of one embodiment of an introducer septum for use in an injection device made in accordance with the invention.
**FIG. 22** is a section view of an injection device made in accordance with the invention including the introducer septum of **FIG. 21****.**
**FIG. 23** is a perspective view of one embodiment of a septum for use in an injection device made in accordance with the invention.
**FIGS. 24A & 24B** are top side and section views, respectively, of one embodiment of a septum for use in an injection device made in accordance with the invention.
**FIG. 25** is a perspective view of one embodiment of an on-body injector for use with an injection device made in accordance with the invention.
**FIG. 26** is a partial perspective view of portions of one embodiment of an on-body injector for use with an injection device made in accordance with the invention.
**FIG. 27** is a partial perspective view of portions of one embodiment of an on-body injector for use with an injection device made in accordance with the invention.
**FIG. 28** is a section view of one embodiment of an injection device and on-body injector for use with an injection device made in accordance with the invention.
**FIG. 29** is a block diagram of one embodiment of an injection device and on-body injector made in accordance with the invention.
**FIG. 30** is a flow chart of a method of use for an on-body injector in accordance with the invention.
**FIG. 31** is a block diagram of one embodiment of an injection device and electronic on-body injector made in accordance with the invention.
**FIGS. 32A-32C** are wave form diagrams of bolus, basal, and basal pump drive signals for an electronic injector made in accordance with the invention.
**FIGS. 33A-33C** are schematic diagrams of a piezoelectric MEMS pump for use in an electronic injector made in accordance with the invention.
**FIGS. 34A-34C** are an exploded perspective view, a partial perspective view, and a partial perspective view of an injection device and electronic on-body injector made in accordance with the invention.
**FIG. 35** is a block diagram of one embodiment of an electronic injector made in accordance with the invention.
**FIGS. 36A-36D** are a perspective view, a top view, a side view, and a partial perspective view of an electronic injector made in accordance with the invention.
**FIGS. 37A-37E** are a perspective view, a top view, a side view, an exploded perspective view, and a partial top view of an electronic injector made in accordance with the invention.
**FIG. 38** is a schematic cross section view of an electronic injector made in accordance with the invention.

### DETAILED DESCRIPTION

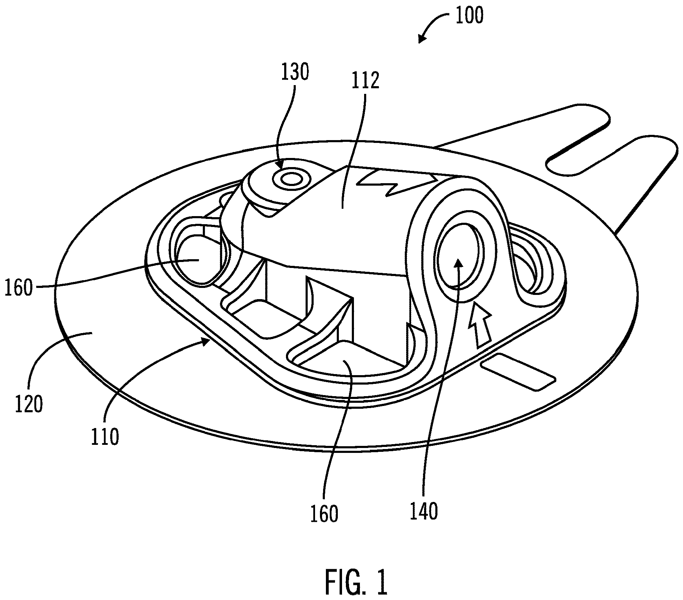

**FIGS. 1-5****,** in which like elements share like reference numbers, are various views of one embodiment of an injection device made in accordance with the invention. The injection device includes an introducer port along an introducer axis and an injection port along an injection axis, with the injection axis being non-collinear with the introducer axis. In this embodiment, the injection axis is at an angle to and intersects with the introducer axis.

**FIG. 1** is a perspective view of the injection device **100** including a body **110** and a patch **120** attached to the body **110.** The patch **120** is operable to adhesively attach the injection device **100** to a patient (not shown). The body **110** has a port face **112,** with an introducer port **130** and an injection port **140** on the port face **112.** The introducer port **130** is used to place a delivery tube subcutaneously in the patient. The injection port **140** is used by the patient to inject a therapeutic agent, which as defined herein can be any liquid such as a liquid including a therapeutic agent, drug, diagnostic agent, or the like. The body **110** also includes cutouts **160.** Those skilled in the art will appreciate that the introducer port **130** can be too small to be effectively used by a patient for injection, but could be used to inject a therapeutic agent, such as a bolus injection using a mechanically attached device, as desired for a particular application.

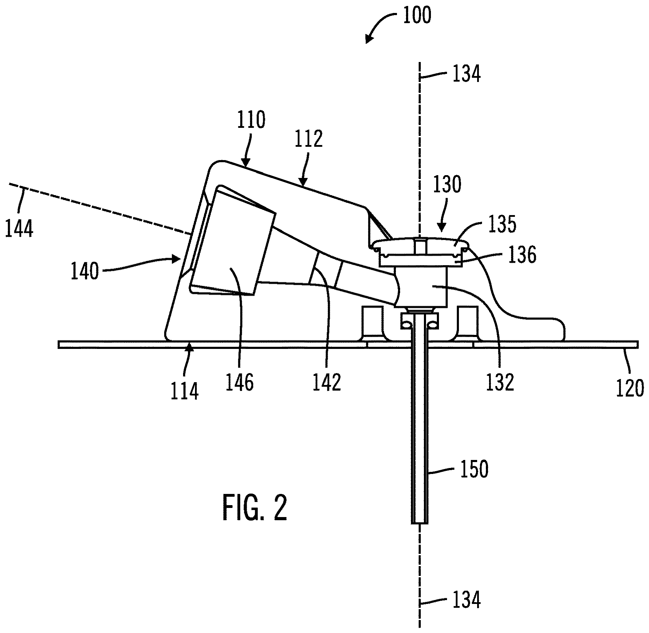

**FIG. 2** is a section view of the injection device **100,** the section bisecting the introducer port **130** and the injection port **140,** and includes the introducer axis **134** and injection axis **144.** An axis as defined herein generally follows the centerline of an associated channel through an associated port. The body **110** has a port face **112** and a patient face **114.** A delivery tube **150** for subcutaneous delivery of the therapeutic agent projects from and is generally perpendicular to the patient face **114.** The delivery tube **150** is operably connected to the introducer port **130** and defines an introducer axis **134** along the introducer channel **132,** the delivery tube **150** being in fluid communication with the injection port **140.** The introducer port **130** includes an introducer channel **132,** with an introducer port cover **135** and an introducer septum **136** disposed in the introducer channel **132.** The injection port **140** includes an injection channel **142** defining an injection axis **144** with an injection septum **146** disposed in the injection channel **142.** In one embodiment, the introducer septum **136** and/or the injection septum **146** is self sealing, such that each of the septums block fluid flow through the septum after a needle has been put through the septum then removed, preventing fluid flow from the port. In this embodiment, the injection axis **144** is at an angle to and intersects with the introducer axis **134.** In one example, the delivery tube **150** is a flexible cannula and a needle hub assembly can be used to place the delivery tube **150** subcutaneously in the patient. In another example, the delivery tube **150** is a rigid needle and the delivery tube **150** can be placed subcutaneously in the patient with or without a needle hub assembly.

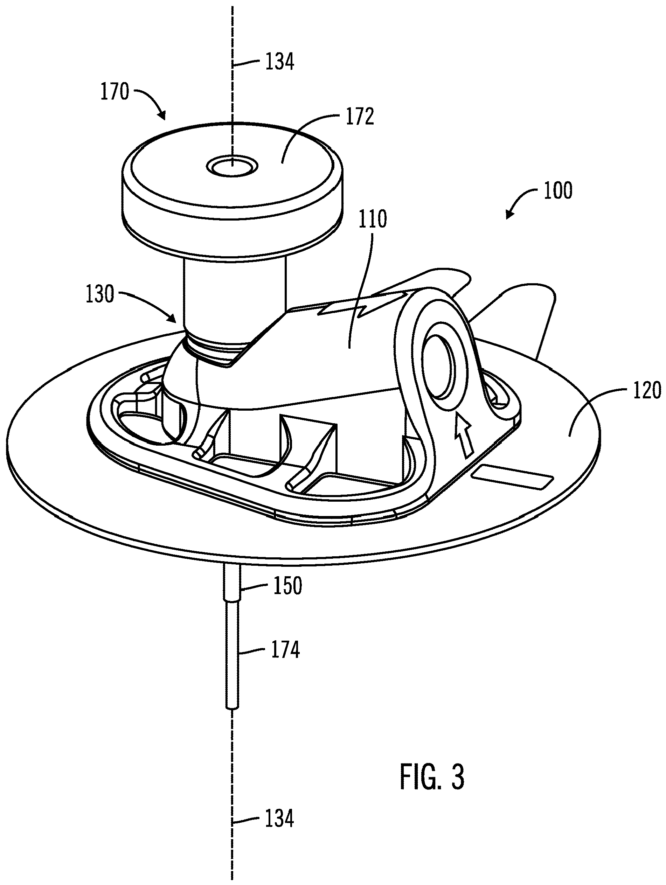

**FIG. 3** is a perspective view of the injection device **100** with a needle hub assembly **170.** The needle hub assembly **170** includes a needle hub **172** and a needle **174** attached to the needle hub **172.** The needle **174** of the needle hub assembly **170** is inserted through the introducer port **130** and through the delivery tube **150** along the introducer axis **134.** The needle hub assembly **170** can be used to add rigidity to the delivery tube **150** during implantation when the delivery tube **150** is a flexible cannula.

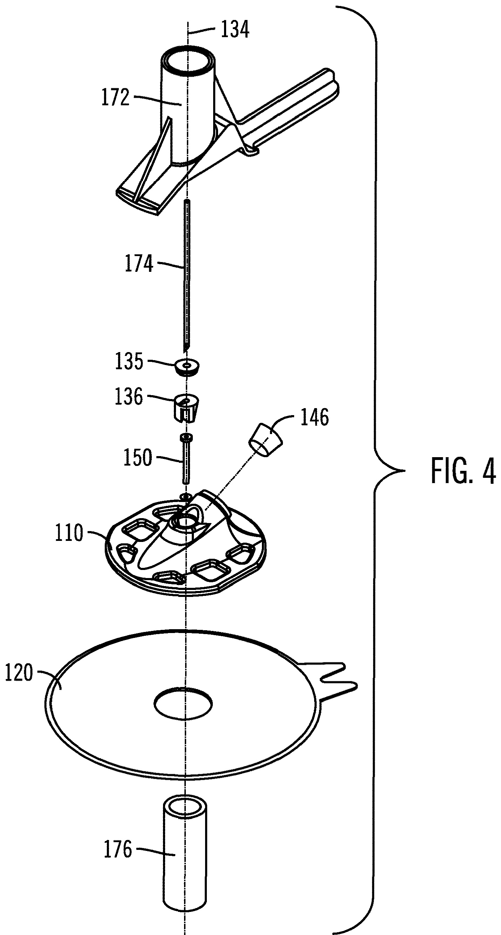

**FIG. 4** is an exploded perspective view of the injection device with a needle hub assembly. A needle guard **176** disposed around the needle **174** can be used to protect the needle **174** and the delivery tube **150** when the injection device and needle hub assembly are assembled for shipping. The various parts of the injection device and needle hub assembly can be connected by interference fit, adhesive, welding, and/or any other method of attachment suitable for a particular application.

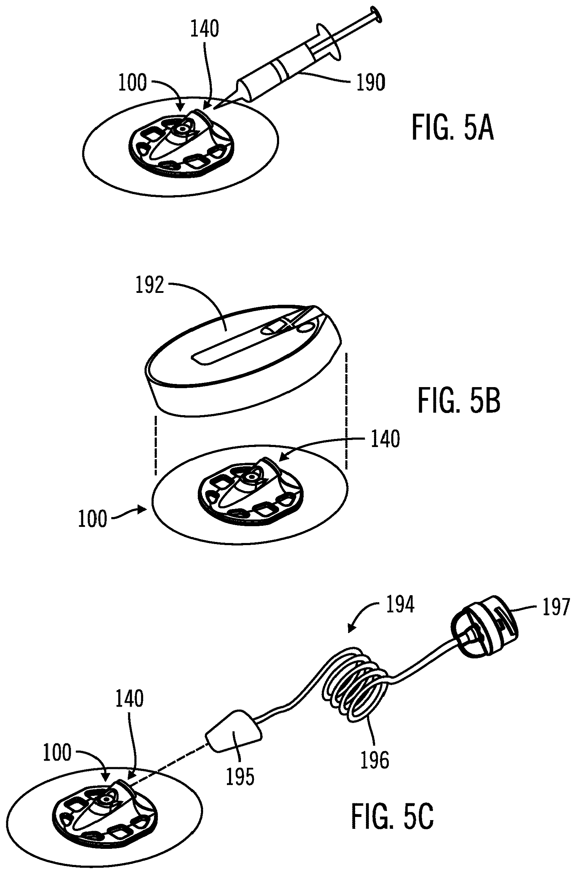

**FIGS. 5A-5C** are perspective views of various applications of the injection device made in accordance with the invention. Referring to **FIG. 5A****,** a syringe **190** can be used to deliver a therapeutic agent through the injection port **140** of the injection device **100.** The syringe can be a conventional syringe, a standard insulin pen, or a needleless syringe. The needle length of a conventional syringe or standard insulin pen can be of any length because the injection axis is non-collinear with the introducer axis, such that a longer needle does not damage the injection device. In one embodiment, the injection port **140** is adapted to be mateable with the syringe **190,** with a socket, fitting, or the like, to increase ease of use. In one example, the injection port **140** is a socket with a socket needle which pierces a foil front end of a needleless syringe when the needleless syringe is seated in the socket. The needleless syringe itself has no needle in this example.

Referring to **FIG. 5B****,** an on-body injector **192** is mateable with the injection port **140** of the injection device **100** and can be used to deliver a therapeutic agent through the injection port **140.** The on-body injector **192** can include a reservoir to hold the therapeutic agent. In one embodiment, the on-body injector **192** can deliver a basal and/or bolus dose of the therapeutic agent.

Referring to **FIG. 5C****,** an extendable tube **194** can be used to deliver a therapeutic agent through the injection port **140.** The extendable tube **194** includes a port connector **195,** a tube **196,** and an external device fitting **197,** all being in fluid communication. The port connector **195** is in fluid communication with the injection port **140** with a needle or mateable fitting to deliver the therapeutic agent through the injection port **140.** The external device fitting **197** is connectable to an external device, such as a wearable insulin pump or an infusion tubing line to a gravity fed container.

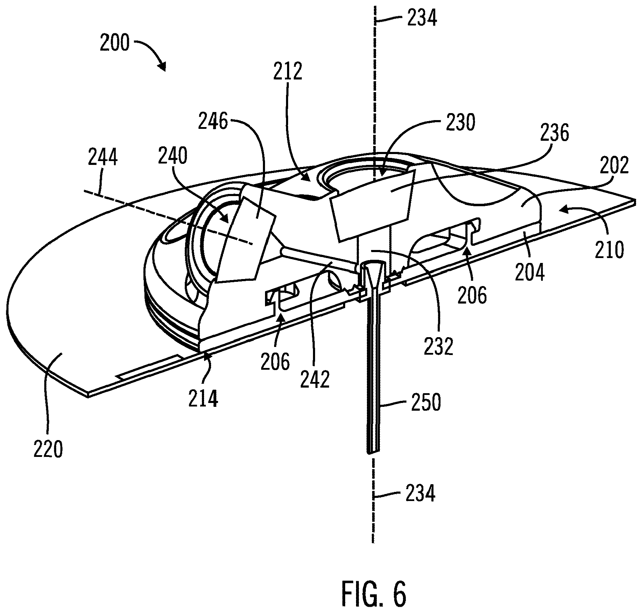

**FIG. 6** is a section perspective view of one embodiment of an injection device made in accordance with the invention. In this embodiment, an upper body portion is rotatable about the introducer axis independent of a lower body portion, so that the injection axis can be positioned at a desired rotary angle regardless of the initial placement of the patch on the patient. This allows the patient to select a rotary position for the injection port that is convenient for injection of the therapeutic agent.

The body of the injection device can have a first body portion including the port face and a second body portion including the patient face, the first body portion and the second body portion being rotatably connected with a flange, the first body portion and the second body portion being independently rotatable about the introducer axis.

The body **210** of the injection device **200** includes an upper body portion **202** and a lower body portion **204.** The upper body portion **202** and lower body portion **204** are rotatably connected with a flange **206** so that the upper body portion **202** and the lower body portion **204** can rotate independently about the introducer axis **234** defined by the delivery tube **250** along the introducer channel **232.** The upper body portion **202** has a port face **212** and the lower body portion **204** has a patient face **214.** A patch **220** is attached to the patient face **214** and is operable to adhesively attach the injection device **100** to a patient (not shown).

The delivery tube **250** for subcutaneous delivery of a therapeutic agent projects from and is generally perpendicular to the patient face **214.** The delivery tube **250** is operably connected to the introducer port **230,** the delivery tube **250** being in fluid communication with the injection port **240.** The introducer port **230** includes an introducer channel **232,** with an introducer septum **236** disposed in the introducer channel **232.** The injection port **240** includes an injection channel **242** defining an injection axis **244** with an injection septum **246** disposed in the injection channel **242.**

The injection axis **244** is non-collinear with the introducer axis **234.** In this embodiment, the injection axis **244** is at an angle to and intersects with the introducer axis **234.** In one example, the delivery tube **250** is a flexible cannula and a needle hub assembly can be used to place the delivery tube **250** subcutaneously in the patient. In another example, the delivery tube **250** is a rigid needle and the delivery tube **250** can be placed subcutaneously in the patient with or without a needle hub assembly.

In operation, the patch **220** is attached to the patient and the delivery tube **250** inserted in the patient for subcutaneous delivery of a therapeutic agent. The injection port **240** in the upper body portion **202** can be rotated about the introducer axis **234** even though the lower body portion **204** is at a fixed position on the patient since the lower body portion **204** is attached to the patient by the patch **220.**

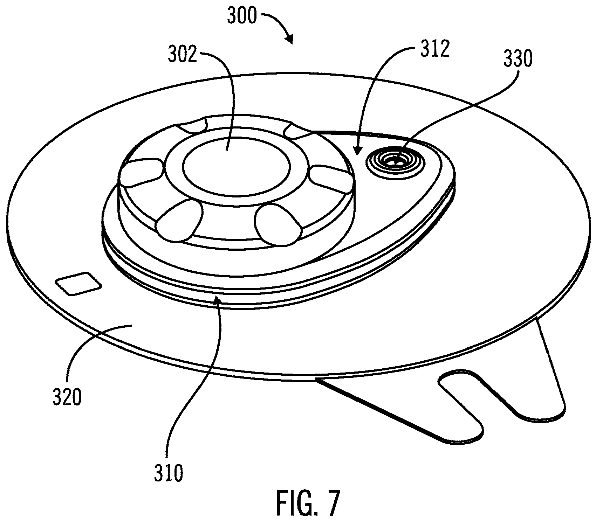

**FIGS. 7-11****,** in which like elements share like reference numbers, are various views of one embodiment of an injection device made in accordance with the invention. The injection device includes an introducer port along an introducer axis and an injection port along an injection axis, with the injection axis being non-collinear with the introducer axis. In this embodiment, the injection axis is parallel to and does not intersect with the introducer axis.

The injection device for delivering a therapeutic agent to a patient can include a body, the body having a patient face and a port face opposite the patient face, the port face having an introducer port including an introducer channel and an injection port including an injection channel, the introducer channel being in fluid communication with the injection channel through a cross channel, the injection channel defining an injection axis; a delivery tube for subcutaneous delivery of the therapeutic agent to the patient, the delivery tube projecting from and being generally perpendicular to the patient face, the delivery tube defining an introducer axis and being in fluid communication with the injection port; and a patch, the patch being attached to the patient face and being operable to adhesively attach to the patient; wherein the injection axis is parallel to the introducer axis.

**FIG. 7** is a perspective view of the injection device **300** including a body **310** and a patch **320** attached to the body **310.** The patch **320** is operable to adhesively attach the injection device **300** to a patient (not shown). The body **310** has a port face **312,** with an introducer port **330** on the port face **312.** The introducer port **330** is used to place a delivery tube subcutaneously in the patient. In this example, an optional injection cap **302** secured to the body **310** to protect an injection port, which is used by the patient to inject a therapeutic agent.

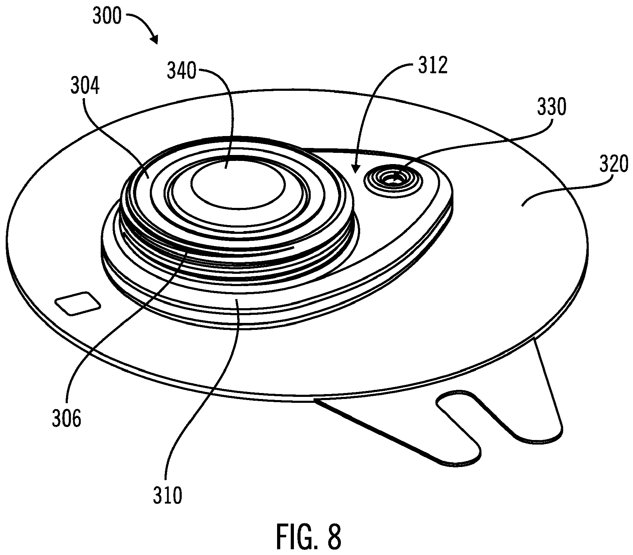

**FIG. 8** is a perspective view of the injection device **300** with the optional injection cap removed to expose the injection port **340.** In this example, the body **312** includes threads **306** to secure the optional injection cap to the body and an optional O-ring **304** to seal the area around the injection port **340** when the optional injection cap is secured to the body.

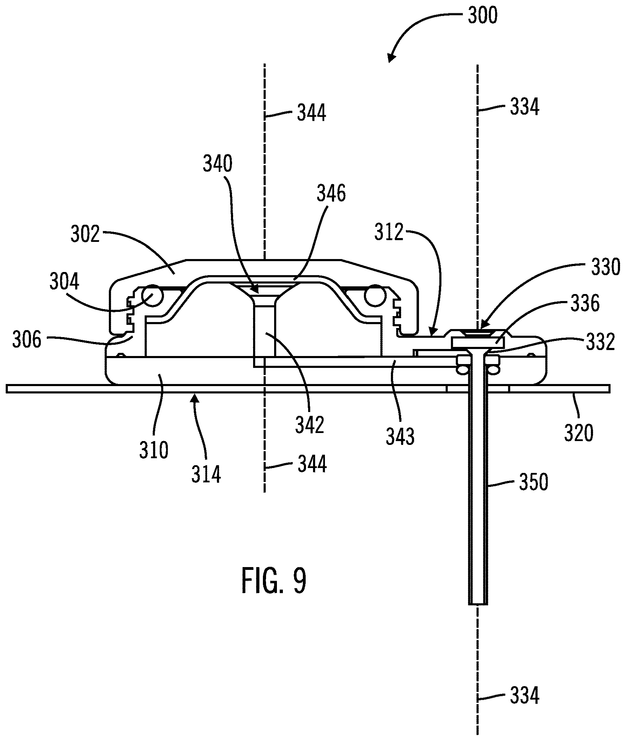

**FIG. 9** is a section view of the injection device **300,** the section bisecting the introducer port **330** and the injection port **340** and including the introducer axis **334** and injection axis **344.** The body **310** has a port face **312** and a patient face **314.** A delivery tube **350** for subcutaneous delivery of the therapeutic agent projects from and is generally perpendicular to the patient face **314.** The delivery tube **350** is operably connected to the introducer port **330** and defines an introducer axis **334** along the introducer channel **332,** the delivery tube **350** being in fluid communication with the injection port **340.** The introducer port **330** includes an introducer channel **332,** with an introducer septum **336** disposed in the introducer channel **332.** The injection port **340** includes an injection channel **342** defining an injection axis **344** with an injection septum **346** disposed over the injection channel **342.** In this embodiment, the injection axis **344** is parallel to and does not intersect with the introducer axis **334.** A cross channel **343** connects the injection channel **342** to the introducer channel **332.** In one example, the delivery tube **350** is a flexible cannula and a needle hub assembly can be used to place the delivery tube **350** subcutaneously in the patient. In another example, the delivery tube **350** is a rigid needle and the delivery tube **350** can be placed subcutaneously in the patient with or without a needle hub assembly.

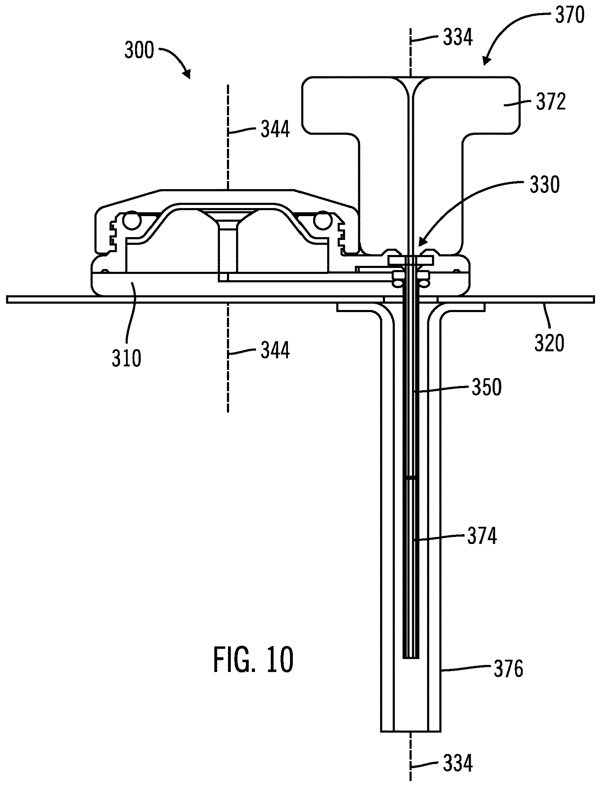

**FIG. 10** is a section view of the injection device **300** with a needle hub assembly **370** and a needle guard **376.** The needle hub assembly **370** includes a needle hub **372** and a needle **374** attached to the needle hub **372.** The needle **374** of the needle hub assembly **370** is inserted through the introducer port **330** and through the delivery tube **350** along the introducer axis **334.** The needle hub assembly **370** can be used to add rigidity to the delivery tube **350** when the delivery tube **350** is a flexible cannula. The needle hub assembly **370** can optionally be used when the delivery tube **350** is a rigid needle. A needle guard **376** disposed around the needle **374** can be used to protect the needle **374** and the delivery tube **350** when the injection device and needle hub assembly are assembled for shipping.

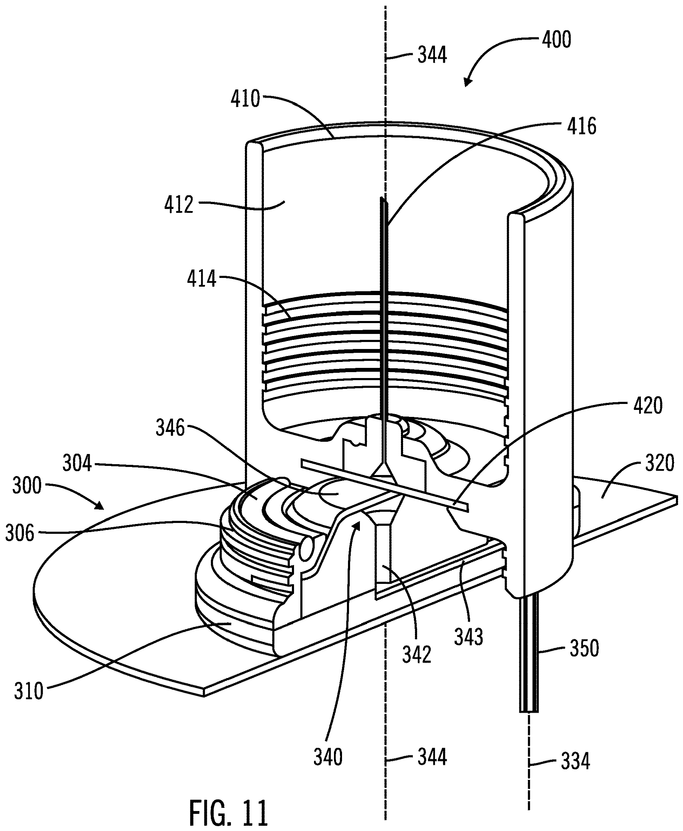

**FIG. 11** is a perspective section view of the injection device with an injection adapter assembly. For clarity of illustration, the cross section cut of the injection device **300** in the illustration bisects the introducer port **330** and the injection port **340,** and includes the introducer axis **334** and injection axis **344.** The cross section cut of the injection adapter assembly **400** in the illustration includes the injection axis **344** and is perpendicular to the section of the injection device **300.** The injection adapter assembly **400** screws onto the injection device **300** using the threads **306** on the body **310** to secure the needleless pen injector to the body **310,** with the O-ring **304** sealing around the interface between the adapter septum **420** and the injector septum **346.** Those skilled in the art will appreciate that the mateable connection securing the needleless pen injector to the body is not limited to threads and can be any mateable connection desired for a particular application.

In this embodiment, the injection adapter assembly **400** is adapted to receive a needleless pen injector (not shown). The adapter body **410** defines a recess **412** adapted to receive a tip of the needleless pen injector. In this example, the needleless pen injector includes threads on its outer diameter complementary to the adapter threads **414** on the inner diameter of the adapter body **410.** The tip of the needleless pen injector is screwed into the recess **412** so that the adapter needle **416** is received in the needleless pen injector, accessing the therapeutic agent contained within the needleless pen injector by piercing a foil on the tip of the needleless pen injector or accessing a pen injector port adapted to receive the adapter needle **416.** With the needleless pen injector secured in the injection adapter assembly **400,** pressure applied to the therapeutic agent enclosed in the needleless pen injector forces the therapeutic agent through the adapter needle **416** and the adapter septum **420** into the injection device **300,** where the therapeutic agent passes through the injector septum **346** into the injection port **340,** through the injection channel **342,** the cross channel **343,** and the delivery tube **350,** and into the patient.

Those skilled in the art will appreciate that a variety of interfaces can be used between the needleless pen injector, the injection adapter assembly **400,** and the injection device **300.** In the embodiment of **FIG. 11****,** the adapter septum **420** and the injector septum **346** are permeable so that the therapeutic agent passes through the adapter septum **420** and the injector septum **346.** The septums can be hydrophilic when used with the needleless pen injector to allow the therapeutic agent to pass through. In another embodiment, the injector septum can include a slit valve operable to open on receiving a stub tube at the tip of the needleless pen injector. In yet another embodiment, the injector septum can include a slit valve which is open by a mechanical lever that pushes open and spread the slit valve when the needleless pen injector is received in the injection adapter assembly. In yet another embodiment, the needleless pen injector is interlocked with the injection adapter assembly so that no therapeutic agent can be dispensed from the needleless pen injector until the needleless pen injector is fully engaged with the injection adapter assembly.

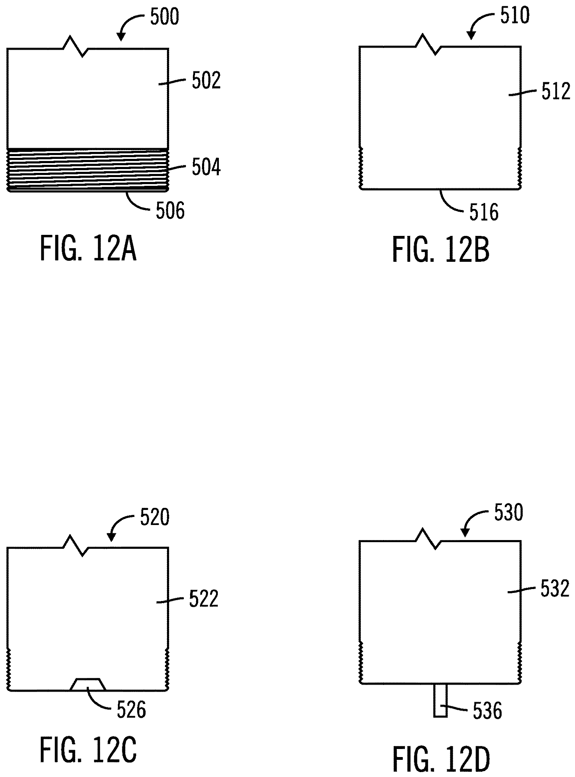

**FIGS. 12A-12D** are various views of needleless pen injectors for use with an injection device made in accordance with the invention. Each of the needleless pen injectors is provided with a manual or automatic pressurization to force the therapeutic agent held within the needleless pen injector into the injection device and patient, once the needleless pen injector has been fully engaged with an injection adapter assembly.

**FIG. 12A** is a side view of the tip of a needleless pen injector **500** having a barrel **502** to contain a therapeutic agent and optional threads **504** for use with an adapter body having threads on the inner diameter. The end **506** of the needleless pen injector **500** can be adapted to accommodate the particular design of an injection adapter assembly for a particular application. **FIG. 12B** is a section view of the tip of a needleless pen injector **510** having a barrel **512** to contain a therapeutic agent and a foil **516** across the end of the needleless pen injector **510.** The foil **516** can be pierced by an adapter needle in the injection adapter assembly (shown in **FIG. 11**) to provide fluid communication between the needleless pen injector **510** and the injection device through the injection adapter assembly. **FIG. 12C** is a section view of the tip of a needleless pen injector **520** having a barrel **522** to contain a therapeutic agent and a pen port **526** at the end of the needleless pen injector **520.** The pen port **526** can receive an adapter needle in the injection adapter assembly (shown in **FIG. 11**) to open the pen port **526** and provide fluid communication between the needleless pen injector **520** and the injection device through the injection adapter assembly. **FIG. 12D** is a section view of the tip of a needleless pen injector **530** having a barrel **532** to contain a therapeutic agent and a stub tube **536** at the end of the needleless pen injector **530.** The stub tube **536** is operable to open a slit valve on the injector septum of the injection device.

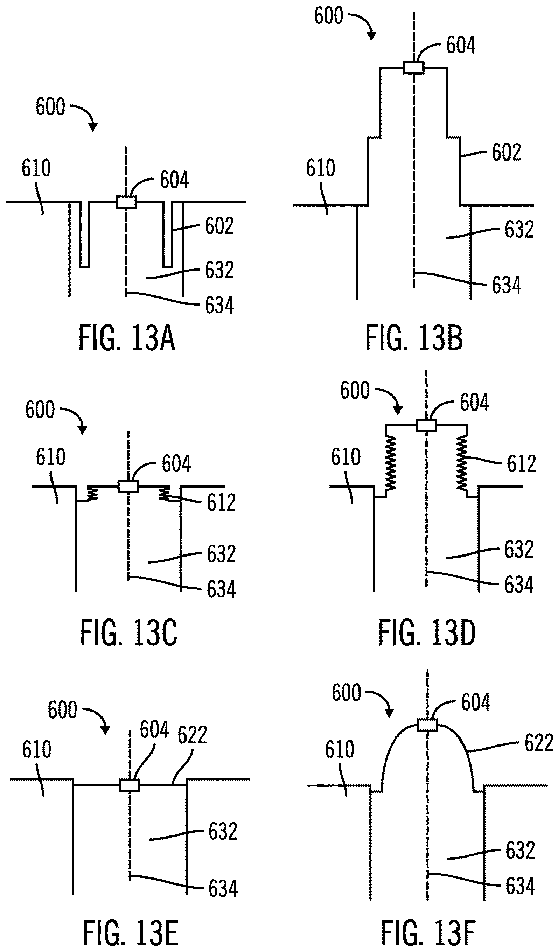

**FIGS. 13A-13F****,** in which like elements share like reference numbers, are section views of pop-up indicator ports for use with an injection device made in accordance with the invention. Because the introducer port and the injection port of the injection device are both in fluid communication with the delivery tube, flow blockage in the delivery tube can cause an increase in pressure at both ports when the patient attempts to inject a therapeutic agent. The flow blockage/pressure increase can be detected by the patient, indicating that the therapeutic agent is not being delivered, with a pop-up indicator port in the port not being used for injection. During injection, the membrane of the pop-up indicator port is close to the body of the injection device under normal conditions, and extends from the body of the injection device when the delivery tube is blocked and the pressure increases above a predetermined pressure.

The pop-up indicator can be disposed in the introducer channel, the pop-up indicator having a normal state when pressure in the introducer channel is normal and an alarm state when pressure in the introducer channel exceeds a predetermined value.

**FIG. 13A & 13B** are section views of a pop-up indicator port **600** with a folded membrane **602** installed as the introducer port. The pop-up indicator port **600** is installed in the introducer channel **632** of the body **610** along the introducer axis **634,** and is in fluid communication with the injection channel. A self-closing port **604** in the membrane **602** allows a needle of a needle hub assembly to pass through the membrane **602** when a needle hub assembly is used to implant the injection device. No self-closing port is required if a needle hub assembly is not used to implant the injection device. Referring to **FIG. 13A****,** the pop-up indicator port **600** is in the normal state with normal pressure in the introducer channel **632,** with the membrane **602** folded on itself. Referring to **FIG. 13B****,** the pop-up indicator port **600** is in the alarm state due to pressure in the introducer channel **632** exceeding a predetermined value. The pressure occurs when a therapeutic agent is being injected into the injection port, which is in fluid communication with the introducer channel **632,** while the delivery tube is blocked. In the alarm state, the membrane **602** unfolds to extend from the body **610.**

**FIG. 13C & 13D** are section views of a pop-up indicator port **600** with an accordion membrane **612** installed as the introducer port. Referring to **FIG. 13C****,** the pop-up indicator port **600** is in the normal state with normal pressure in the introducer channel **632,** with the membrane **612** pleated like an accordion. Referring to **FIG. 13D****,** the pop-up indicator port **600** is in the alarm state due to pressure in the introducer channel **632** exceeding a predetermined value. The pressure occurs when a therapeutic agent is being injected into the injection port, which is in fluid communication with the introducer channel **632,** while the delivery tube is blocked. In the alarm state, the membrane **612** uncompresses the pleats to extend from the body **610.**

**FIG. 13E & 13F** are section views of a pop-up indicator port **600** with a deformable membrane **622** installed as the introducer port. Referring to **FIG. 13E****,** the pop-up indicator port **600** is in the normal state with normal pressure in the introducer channel **632,** with the membrane **622** extending across the introducer channel **632.** Referring to **FIG. 13F****,** the pop-up indicator port **600** is in the alarm state due to pressure in the introducer channel **632** exceeding a predetermined value. The pressure occurs when a therapeutic agent is being injected into the injection port, which is in fluid communication with the introducer channel **632,** while the delivery tube is blocked. In the alarm state, the material of the membrane **622** deforms under pressure to extend from the body **610.** In another embodiment, the material of the membrane **622** can deforms sufficiently to allow the therapeutic agent to leak through the membrane **622,** providing additional indication of the high pressure and delivery tube blockage.

Those skilled in the art will appreciate that the material and dimensions of the parts of the membrane (folds and/or pleats) can be selected as desired for a particular application. In one embodiment, the material is resilient, so that the membrane returns to the normal state after being in the alarm state. In another embodiment, the material is deformable so that the membrane remains extending from the body after the pressure is relieved and the alarm state clears. The extended membrane reminds the patient of the delivery tube blockage and the need to replace the injection device. Exemplary materials for the membrane include silicone rubber or the like.

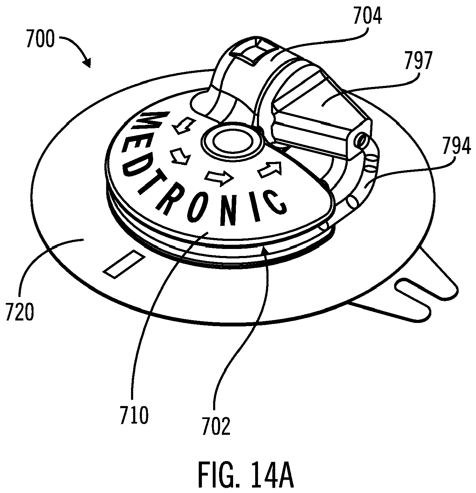

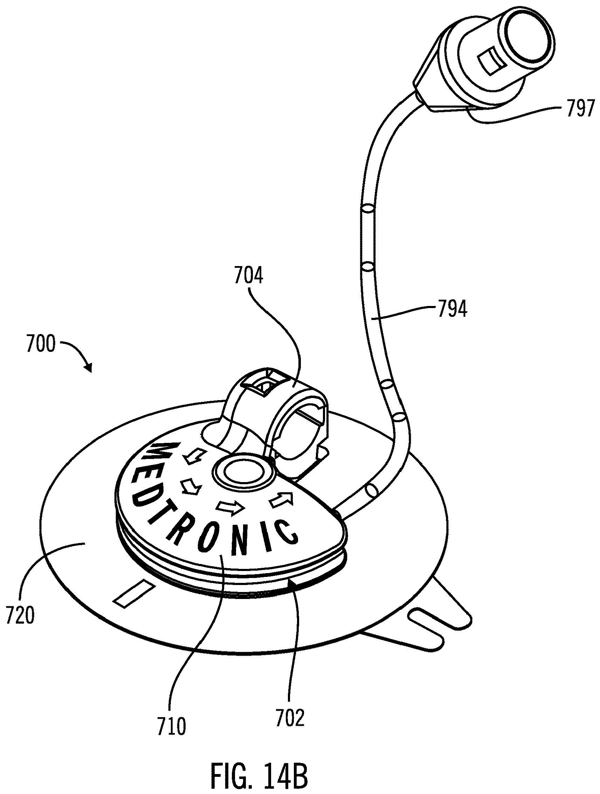

**FIGS. 14A** **&** **14B****,** in which like elements share like reference numbers, are perspective views of one embodiment of an injection device made in accordance with the invention. In this embodiment, the injection device includes a tube with an external device fitting, so that the injection device can be placed in a remote location and attached to an injection pump.

**FIG. 14A** is a perspective view of the injection device **700** in a stored configuration, the injection device **700** including a body **710** and a patch **720** attached to the body **710.** The patch **720** is operable to adhesively attach the injection device **700** to a patient (not shown). The body **710** has a groove **702** around its outer circumference operable to receive tube **794** in the stored configuration. One end of the tube **794** is in fluid communication with an injection port (not shown) of the injection device **700** to deliver a therapeutic agent into the body of a patient. The other end of the tube **794** is in fluid communication with the external device fitting **797,** which can be extended to a convenient location when the injection device **700** is in a difficult to access location or which can be connected to an injection pump (not shown). In this example, the body **710** includes a fitting receiver **704** operable to receive and store the external device fitting **797** when the injection device **700** is in the stored configuration with the tube **794** wrapped around the body **710.** **FIG. 14B** is a perspective view of the injection device **700** in a deployed configuration, with the external device fitting **797** uncoupled from the fitting receiver **704** and the tube **794** uncoiled from the groove **702** in the body **710.** In operation, the injection device **700** can be placed on a remote location on the body of the patient, such as a remote location not normally accessible for injection by conventional means, and the tube **794** extended to allow convenient connection to an injection pump.

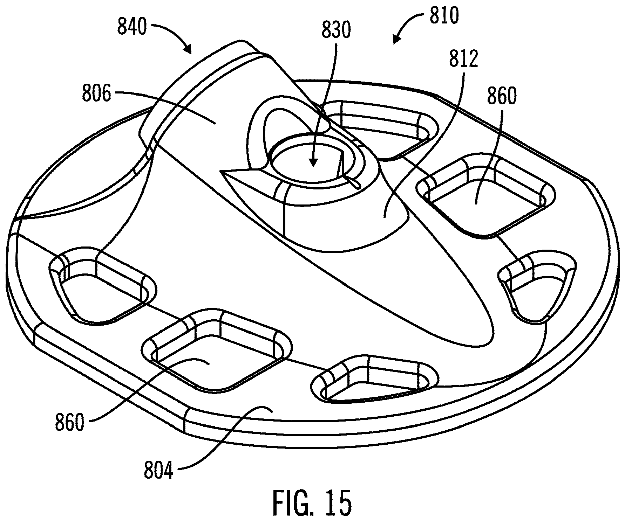

**FIGS. 15-20****,** in which like elements share like reference numbers, are various views of one embodiment of a body for an injection device made in accordance with the invention. The body includes cutouts to provide inspection and ventilation at the attachment point of the injection device to the patient.

The single piece body for an injection device can include a planar deck having a patient face, the planar deck having cutouts around and through the planar deck, the planar deck including a delivery tube port on the patient face; a port segment attached opposite the patient face of the planar deck, the port segment including an introducer port including an introducer channel and an injection port including an injection channel the introducer channel being in fluid communication with the injection channel and the delivery tube port; and attachment projections protruding from the patient face. In one embodiment, the attachment projections are operable for plastic welding.

The single piece body can be used with an injection device for delivering a therapeutic agent to a patient including the single piece body. The injection device further includes a delivery tube for subcutaneous delivery of the therapeutic agent to the patient, the delivery tube projecting from and being generally perpendicular to the patient face, the delivery tube being in fluid communication with the injection port; and a patch, the patch being plastically welded to the attachment projections and being operable to adhesively attach to the patient.

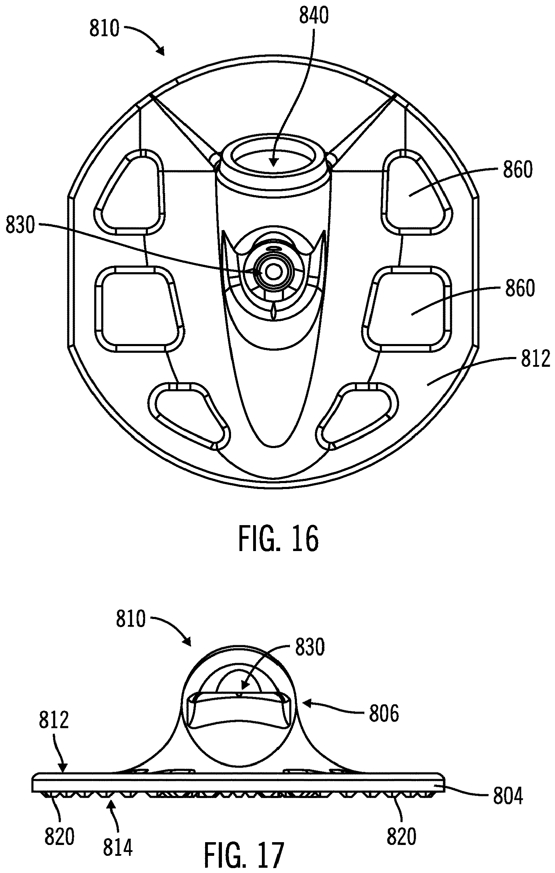

**FIGS. 15** **&** **16** are a front perspective view and a top side view, respectively, of a body **810** including a port face **812.** The port face **812** includes an introducer port **830** and an injection port **840.** The body **810** has a generally planar deck **804** with cutouts **860** spaced around and passing through the planar deck **804.** The body **810** also has a port segment **806** rising above the planar deck **804** and including the introducer port **830** and an injection port **840.** The body **810** is a single piece body, which is defined herein as a body formed as a single piece and is not a group of separate pieces assembled to form the body.

**FIG. 17** is a left side view of the body **810.** The patient face **814** is opposite the port face **812** on the planar deck **804** and is operable to connect the body **810** to a patch (not shown) to adhesively attach the injection device to a patient. In this embodiment, the patient face **814** of the planar deck **804** includes a number of attachment projections **820** (in this example, the attachment projections **820** being bumps) protruding from the planar deck **804** to allow a patch to be plastically welded to the body **810.** Those skilled in the art will appreciate that different attachment projections, such as truncated pyramids, bumps, radial lines, concentric rings, or the like, can be selected as desired for a particular application. In yet another embodiment, the patch can be attached to the body **810** with an adhesive.

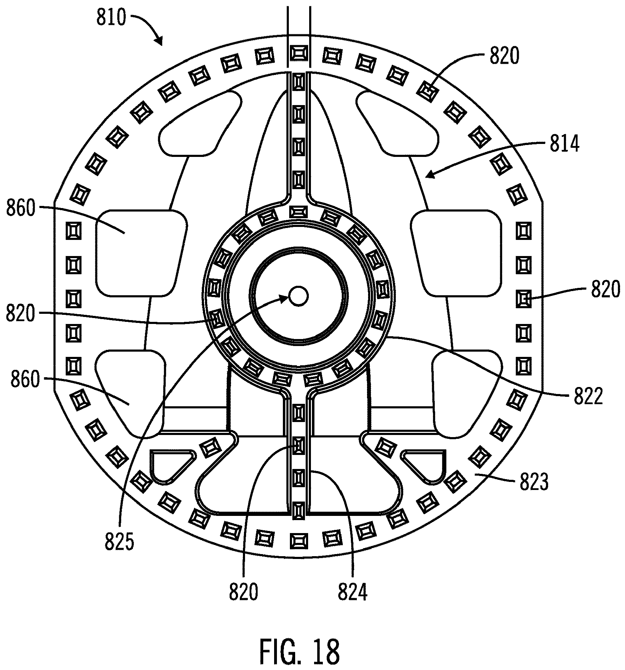

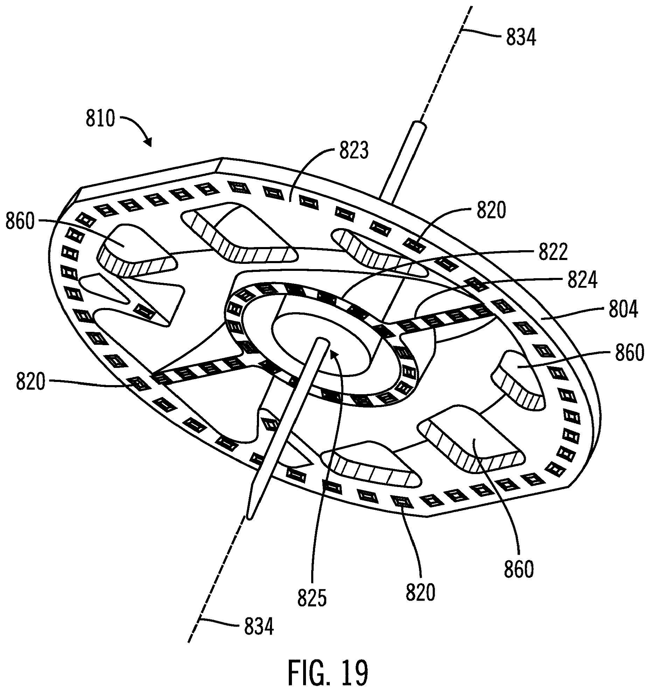

**FIGS. 18** **&** **19** are a bottom side view and a bottom perspective view, respectively, of the body **810.** The attachment projections **820** are arranged around the outer circumference **823** of the patient face **814,** around an inner circle **822** about a delivery tube port **825** on the introducer axis **834,** and along diameter segments **824** between the outer circumference **823** and the inner circle **822** which follow the length of the port segment. In this example, the attachment projections **820** are truncated pyramids.

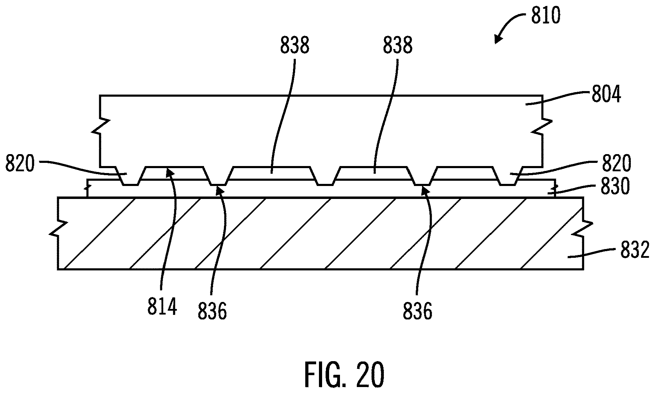

**FIG. 20** is a section view of the planar deck **804** of the body **810** along the outer circumference through the attachment projections **820.** In this example, the body **810** is plastically welded to a patch **830,** which is attached to the skin **832** of a patient. The attachment projections **820** are deformed from the truncated pyramid to a flattened, rounded shape from welding the attachment projections **820** to the patch **830** at each fixation point **836.** In this example, the tips of the attachment projections **820** are welded into the patch **830,** i.e., the tips of the attachment projections rest below the surface of the patch at the fixation points **836** where the attachment projections **820** join the patch **830.** In cross section through adjacent attachment projections **820,** the patient face **814** and the patch **830** define a ventilation gap **838** to provide ventilation and air circulation between the planar deck **804** and the patch **830,** cooling the skin **832** across the patch **830** from the ventilation gap **838.**

Those skilled in the art will appreciate that the design of the patch **830** can be selected as desired for a particular application. The patch can be made of any biocompatible material with biocompatible adhesive operable to hold the weight of the injection device to the skin for a predetermined number of days. The patch design also needs to account for ventilation and circulation between the patch and the skin. In one example, the patch is a continuous sheet of adhesive material. In another example, the patch is a mesh sheet of adhesive material including perforations. In yet another example, the patch is a continuous sheet of adhesive material with holes cut into the continuous sheet. The holes can align with features of the body of the injection device, such as the cutouts, as desired. The holes can optionally be the same size as the cutouts. In yet another example, the patch is a continuous sheet of adhesive material with holes cut into the continuous sheet, and mesh applied across the holes. In yet another example, the patch can be made of a transparent material to allow the condition of the skin around and below the injection device to be monitored. In one example, adhesive patches are constructed of pressure sensitive acrylic-based adhesives with non-woven polyester backings.

Those skilled in the art will further appreciate that the design of the body of the injection device can be selected as desired for a particular application. In one example, the number and position of the cutouts in the planar deck can be selected to provide ventilation to the skin while maintaining sufficient rigidity for the planar deck. In another example, the number and position of the cutouts can be selected to allow observation of the condition of the skin around and below the injection device. In yet another example, the body of the injection device can be made of a transparent material to allow the condition of the skin around and below the injection device to be monitored. This is particularly useful when the patch includes holes or is made from a transparent material. Exemplary materials for the body of the injection device include polycarbonate, acrylic, or the like. In one embodiment, one or more optical elements can be molded into the body of the injection device to magnify the area or areas of interest.

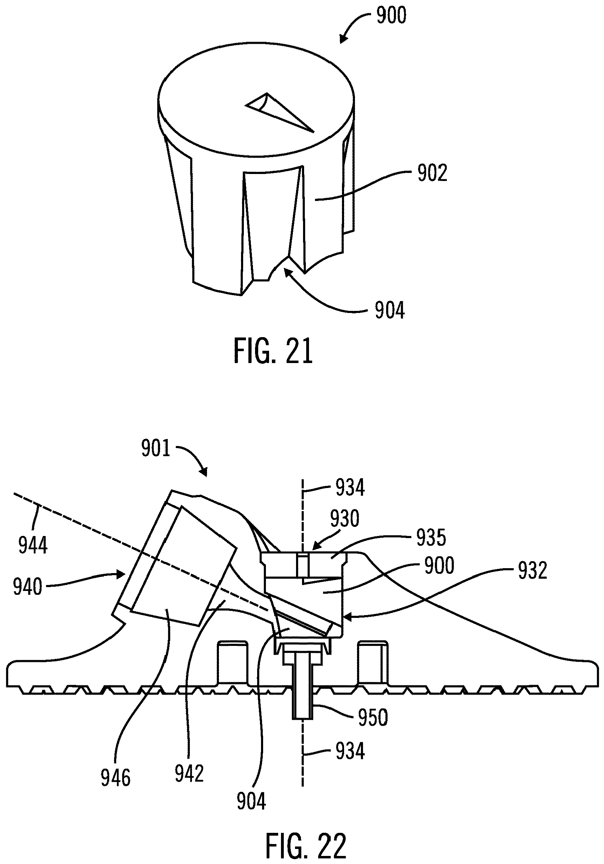

**FIGS. 21-24** are various embodiments of septums for use in an injection device. The septums can be disposed in the injection device channels. In one embodiment, the septum is self sealing to block fluid flow through the septum after a needle has been put through the septum then removed, preventing fluid flow through the port connected to the channel.

**FIG. 21** is a perspective view of one embodiment of an introducer septum for use in an injection device made in accordance with the invention. In this embodiment, the introducer septum is irregular-shaped, i.e., the introducer septum has an irregular shape. The introducer septum **900** includes a number of legs **902** to secure the introducer septum **900** in the introducer channel.

**FIG. 22** is a section view of an injection device made in accordance with the invention including the introducer septum of **FIG. 20****.** The section bisects the introducer port **930** and the injection port **940,** and includes the introducer axis **934** and injection axis **944.** The delivery tube **950** is operably connected to the introducer port **930** and defines an introducer axis **934,** the delivery tube **950** being in fluid communication with the injection port **940.** The introducer port **930** includes an introducer channel **932,** with an introducer port cover **935** and the introducer septum **900** disposed in the introducer channel **932.** The introducer septum **900** is secured in the introducer channel **932** of the injection device **901** by legs **902.** The injection port **940** includes an injection channel **942** defining an injection axis **944** with an injection septum **946** disposed in the injection channel **942.** The injection channel **942** is in fluid communication with the delivery tube **950** through a septum connection channel **904** in the introducer septum **900.** The introducer septum **900** both connects the injection port **940** to the delivery tube **950** and fills extra space within the introducer channel **932** to avoid an unnecessary amount of therapeutic agent from collecting in the introducer channel **932.**

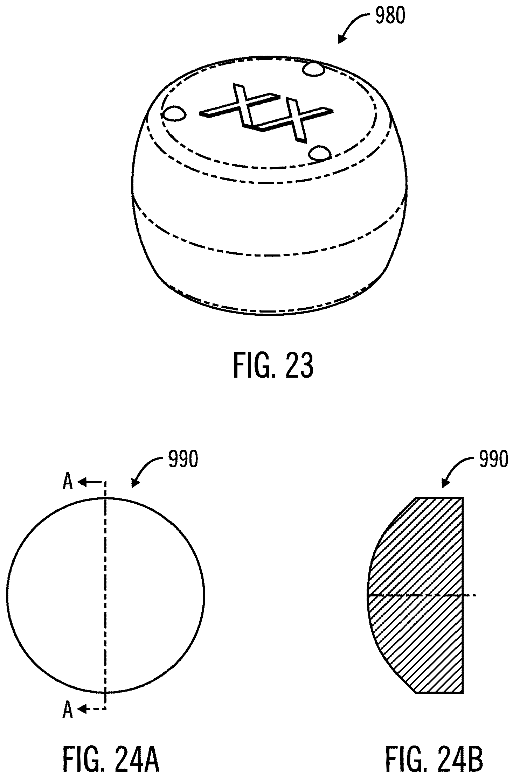

**FIG. 23** is a perspective view of one embodiment of a septum for use in an injection device made in accordance with the invention. In this embodiment, the septum is barrel-shaped. The barrel-shape septum **980** can be used as an introducer septum or an injection septum as desired for a particular application.

**FIGS. 24A & 24B** are top side and A-A section views, respectively, of one embodiment of a septum for use in an injection device made in accordance with the invention. In this embodiment, the septum is dome-shaped. The dome septum **990** can be used as an introducer septum or an injection septum as desired for a particular application.

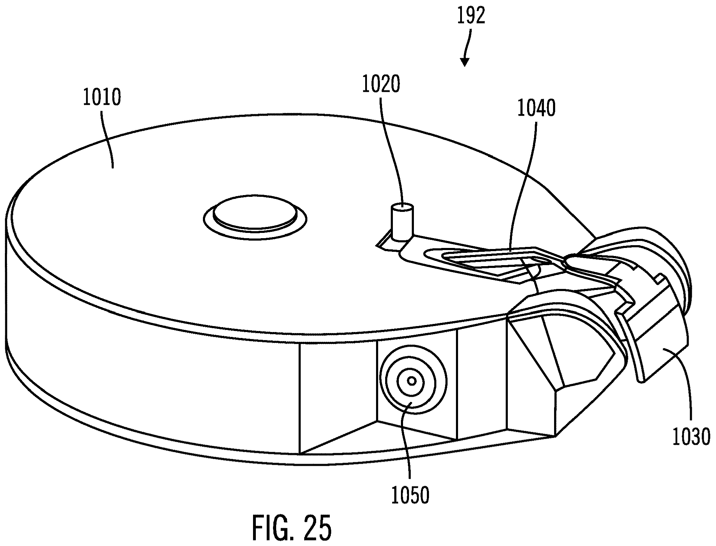

**FIGS. 25-30** illustrate an on-body injector for use with an injection device made in accordance with the invention. Referring to **FIG. 5B****,** an on-body injector **192** is mateable with the injection port **140** of the injection device **100** and can be used to deliver a therapeutic agent through the injection port **140.** The on-body injector **192** can include a reservoir to hold the therapeutic agent. In one embodiment, the on-body injector **192** can deliver a basal and/or bolus dose of the therapeutic agent.

**FIG. 25** is a perspective view of one embodiment of an on-body injector for use with an injection device made in accordance with the invention. The on-body injector **192** includes a housing **1010** to contain the internal components of the on-body injector, a lock **1020** to secure the on-body injector **192** to the injection device, and a fill port **1050** for filling or refilling the on-body injector **192** with a therapeutic agent for bolus and/or basal injection.

The on-body injector **192** also includes a button **1030** which can be used to administer a bolus injection. A gap in the bolus injection flow path prevents bolus injection unless the button **1030** is depressed. The button **1030** is operably connected to the bolus injection needle **1040** to slide the bolus injection needle **1040** along the injection axis. When the button **1030** is depressed, the button **1030** advances the bolus injection needle tip to close a gap between the bolus injection needle tip and the injection port of the injection device and to form an injection flow path to deliver a bolus injection to the patient. The button **1030** also advances a plunger through the bolus reservoir to deliver a predetermined bolus volume to the patient through the injection flow path once the gap is closed and the injection flow path is complete. Those skilled in the art will appreciate that the button **1030** as defined herein can be any mechanism or combination of mechanisms operable to advance the bolus injection needle through the gap and deliver the bolus injection. In one example, the button slides in a track or similar guiding geometry. In another example, the button is a lever that moves the bolus injection needle and plunger. Those skilled in the art will appreciate that the button can be activated by secondary devices, such as solenoids, motors, pneumatic activators, or the like.

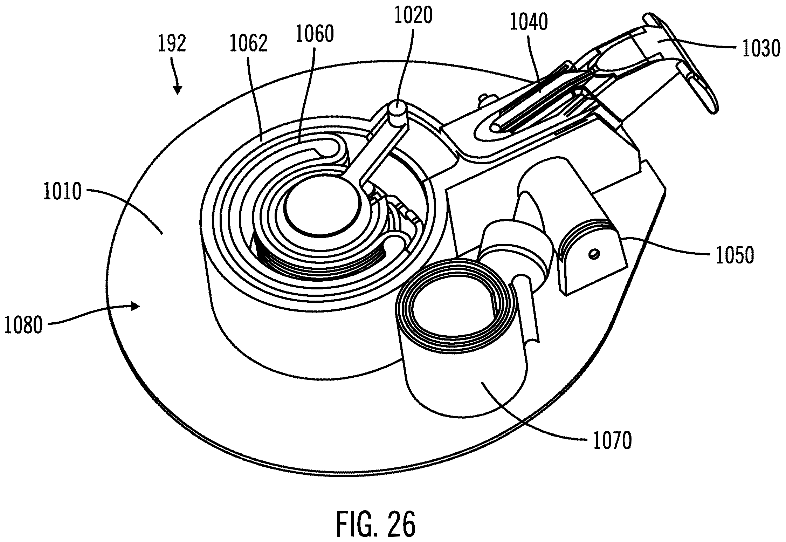

**FIG. 26****,** in which like elements share like reference numbers with **FIG. 25****,** is a partial perspective view of portions of one embodiment of an on-body injector for use with an injection device made in accordance with the invention. In **FIG. 26****,** the top portion of **the** housing **1010** has been removed to reveal the interior components. In this embodiment, basal injection is provided by a pressurized reservoir **1070,** which is at least partially filled with a therapeutic agent. The pressurized reservoir **1070** in this example is a spring coil that at least partially uncoils within an interior track **1080** within the housing **1010** when the pressurized reservoir **1070** is pressurized, i.e., when the pressurized reservoir is at least partially filled with a therapeutic agent. The pressurized reservoir **1070** can be filled through the fill port **1050.** For basal injection, the therapeutic agent is delivered through the introducer port of the injection device, which is in fluid communication with the delivery tube of the injection device. The therapeutic agent passes from the pressurized reservoir **1070,** through the flow restrictor **1060,** and into the patient through the injection device. The flow restrictor **1060** in this example is tubing having a length and interior diameter selected to provide a desired pressure drop, supported by tubing support structure **1062.** Those skilled in the art will appreciate that the flow restrictor can be any device providing a pressure drop between the pressurized reservoir and the delivery tube as desired for a particular application. In another example, the flow restrictor can be an orifice. In another example, the flow restrictor can be a bypass channel that re-directs access to medication. In one embodiment, the flow restrictor can be selected to provide a predetermined basal flow rate, such as a basal flow rate of 20 units of insulin per 24 hours, 30 units of insulin per 24 hours, or 40 units of insulin per 24 hours. Those skilled in the art will further appreciate that the pressurized reservoir can be any device pressurizing the therapeutic agent as desired for a particular application. In other examples, the pressurized reservoir can be an elastic bladder, a spring-loaded inelastic bladder, a fluid (gas or liquid) pressurized bladder, or the like.

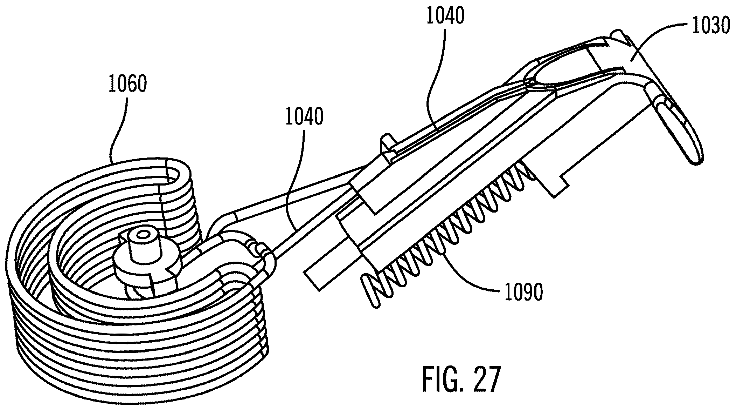

**FIG. 27****,** in which like elements share like reference numbers with **FIGS. 25** **&** 26, is a partial perspective view of portions of one embodiment of an on-body injector for use with an injection device made in accordance with the invention. A button spring **1090** provides a bias force to the button **1030,** to bias the bolus injection needle **1040** away from the injection port of the injection device and provide the gap between the bolus injection needle tip and the injection port when the button is not depressed.

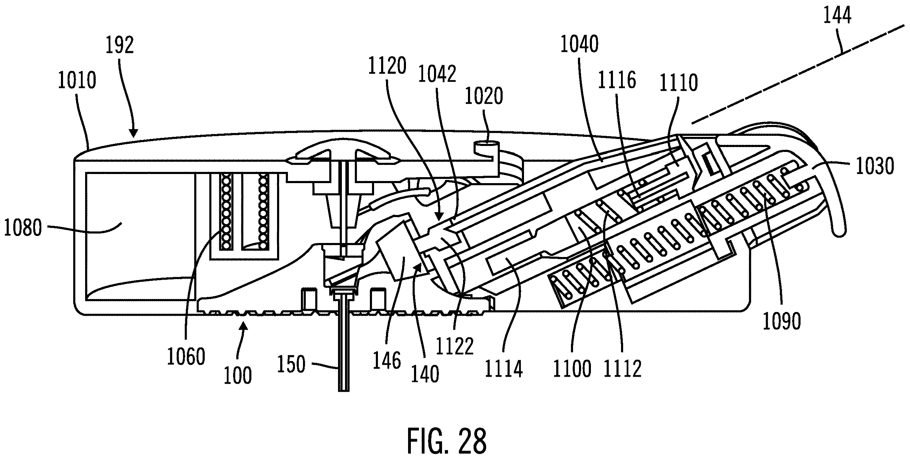

**FIG. 28****,** in which like elements share like reference numbers with **FIGS. 25-****27,** is a section view of one embodiment of an injection device and on-body injector for use with an injection device made in accordance with the invention. **FIG. 28** illustrates the cross-section of the on-body injector **192** and the injection device **100** divided along the bolus injection needle **1040.**

The bolus injection needle tip **1042** is aligned with the injection septum **146** of the injection port **140** along the injection axis **144.** The button spring **1090** biases the bolus injection needle tip **1042** away from the injection septum **146** to create a gap **1120** between the bolus injection needle tip **1042** and the injection port **140** when the button **1030** is not depressed. When the button **1030** is depressed, the bolus injection needle **1040** slides along the injection axis **144,** closing the gap **1120** and inserting the bolus injection needle tip **1042** through the injection septum **146.** In this example, the bolus injection needle tip **1042** also passes through a needle tip septum **1122.** Once the bolus injection needle tip **1042** has passed through the injection septum **146,** and injection flow path for bolus injection is formed from the bolus reservoir **1100,** through the bolus injection needle **1040,** through the delivery tube **150,** and into the patient. When the button **1030** is released, the bolus injection needle **1040** slides back to the initial position and the gap **1120** is restored. The bolus injection needle tip **1042** is always embedded within a septum (the needle tip septum **1122** or the injection septum **146**) to keep the bolus injection needle tip **1042** clean, and to keep the bolus injection needle tip **1042** capped so no therapeutic agent will leak out of the bolus injection needle tip **1042.**

The on-body injector **192** includes a bolus reservoir **1100** with a plunger **1110** slideably disposed within the bolus reservoir **1100** and a bolus stop **1114** fixed at one end of the bolus reservoir **1100.** A bolus spring **1112** biases the plunger **1110** away from the bolus stop **1114.** The plunger **1110 is** coupled to the button **1030** so that the plunger **1110** slides through the bolus reservoir **1100** when the button **1030** is depressed. The plunger **1110** includes a central passage **1116** to allow fluid from the bolus reservoir **1100** to flow through the plunger **1110** into the bolus injection needle **1040** and to the patient through the bolus injection needle **1040.** The volume of the bolus reservoir **1100** can be selected to provide a predetermined bolus volume to the patient. In one embodiment, the predetermined bolus volume is 2 units of insulin.

When the plunger **1110** reaches a final position at the end of the bolus reservoir **1100** and contacts the bolus stop **1114,** the bolus stop **1114** blocks the central passage **1116** in the plunger **1110.** Thus, the plunger **1110** blocks the injection flow path after the predetermined bolus volume has been delivered. This prevents additional undesired delivery of a therapeutic agent should the injection flow path remains in place from a failure, such as the bolus injection needle **1040** becoming stuck in the injection port **140** of the injection device **100,** a failure of the button **1030** or related mechanism, or the like.

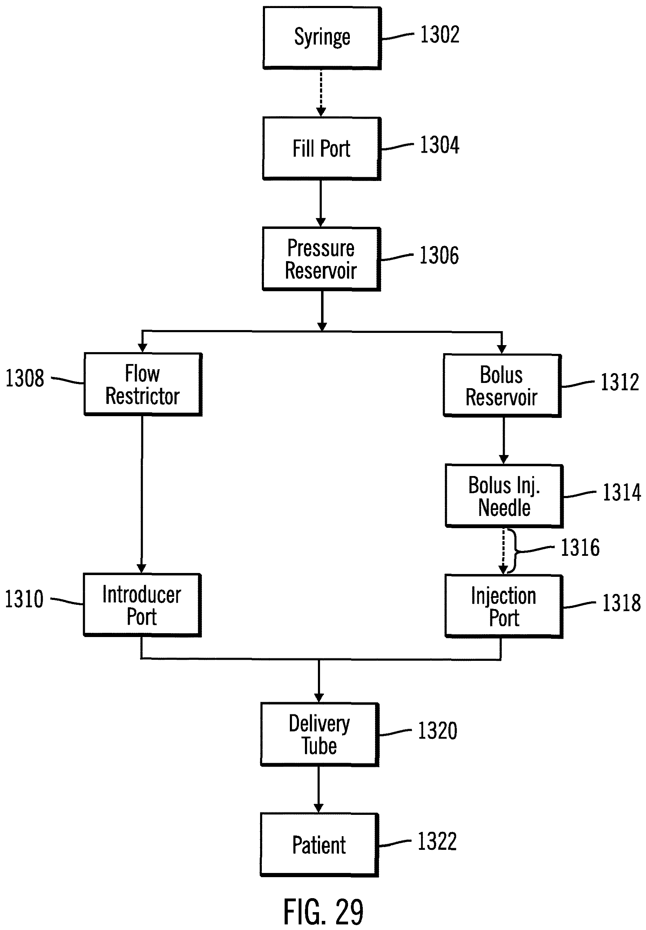

**FIG. 29** is a block diagram of one embodiment of an injection device and on-body injector made in accordance with the invention. **FIG. 29** illustrates the flow paths through the injection device and on-body injector, which can be used for a bolus injection and/or a basal injection.

Both the bolus injection and basal injection flow paths include an optional syringe **1302,** an optional fill port **1304,** a pressure reservoir **1306,** a delivery tube **1320,** supplying the patient **1322.** The basal injection flow path also includes a flow restrictor **1308** and an introducer port **1310** between the pressure reservoir **1306** and the delivery tube **1320.** The bolus injection flow path also includes a bolus reservoir **1312,** a bolus injection needle **1314,** a gap **1316,** an injection port 1318 between the pressure reservoir **1306** and the delivery tube **1320.**

The optional syringe **1302** can be inserted in the optional fill port **1304** to fill or refill the pressure reservoir **1306.** As illustrated by the dashed line between the optional syringe **1302** and the optional fill port **1304,** the optional syringe **1302** is not permanently attached to the optional fill port **1304** and can be removed. In one embodiment, the pressure reservoir **1306** can be pre-filled with a therapeutic agent when the on-body injector is delivered to the patient, so that the fill port **1304** is used for refilling the pressure reservoir **1306.** In another embodiment, the pressure reservoir **1306** is empty when the on-body injector is delivered to the patient, so that the fill port **1304** is used for initially filling the pressure reservoir **1306.** In yet another embodiment, the optional fill port **1304** is omitted and the on-body injector is a single use device with the pressure reservoir **1306** pre-filled with a therapeutic agent. Those skilled in the art will appreciate that the optional syringe **1302** can be any device operable to deliver a therapeutic agent into the fill port **1304** as desired for a particular application.

For basal injection, the pressure reservoir **1306** of the on-body injector provides the therapeutic agent through the flow restrictor **1308** of the on-body injector to the introducer port **1310** of the injection device. The delivery tube **1320** of the injection device applies the therapeutic agent to the patient **1322.** The flow restrictor **1308** can be any device providing a pressure drop between the pressurized reservoir and the delivery tube as desired for a particular application. In one example, the flow restrictor can be tubing having a length and interior diameter selected to provide a desired pressure drop. In another example, the flow restrictor can be an orifice. In another example, the flow restrictor can be a bypass channel that re-directs access to medication. In one embodiment, the flow restrictor can be selected to provide a predetermined basal flow rate, such as a basal flow rate of 20 units of insulin per 24 hours, 30 units of insulin per 24 hours, or 40 units of insulin per 24 hours.

For bolus injection, the pressure reservoir **1306** of the on-body injector fills the bolus reservoir **1312** of the on-body injector with therapeutic agent. The on-body injector delivers a predetermined bolus volume (the volume of the pressure reservoir **1306)** when the patient depresses a button. When the button is depressed, the tip of the bolus injection needle **1314** closes the gap **1316** between the bolus injection needle **1314** of the on-body injector and enters the injection port **1318** of the injection device to complete the bolus injection flow path. The gap **1316** as illustrated by the dashed lines between the bolus injection needle **1314** of the on-body injector and the injection port **1318** of the injection device is present when the button is not depressed to prevent bolus injection unless the button is depressed. Depressing the button also delivers the therapeutic agent from the bolus reservoir **1312,** through the bolus injection needle **1314,** through the injection port **1318,** through the delivery tube **1320,** and into the patient **1322.** In one embodiment, the predetermined bolus volume is 2 units of insulin. The bolus injection flow path can optionally include a flow blocker (such as the bolus stop **1114** of **FIG. 28****,** for example) which blocks the bolus injection flow path after the predetermined bolus volume has been delivered.

Referring to **FIG. 29****,** for one embodiment of an on-body injector for a bolus injection, the injection device has an injection port **1318** in fluid communication with a delivery tube **1320** with the injection port **1318** lying on an injection axis. The on-body injector includes a bolus reservoir **1312;** a bolus injection needle **1314** in fluid communication with the bolus reservoir **1312,** the bolus injection needle **1314** having a bolus injection needle tip aligned with the injection port **1318,** the bolus injection needle **1314** being slideably biased away from the injection port to define a gap **1316** between the bolus injection needle tip and the injection port **1318;** and a button operably connected to the bolus injection needle **1314** to slide the bolus injection needle **1314** along the injection axis. The button is operable to advance the bolus injection needle tip to close the gap **1316** and advance the bolus injection needle tip into the injection port **1318** to form an injection flow path from the bolus reservoir **1312,** through the bolus injection needle **1314,** through the delivery tube **1320,** and into the patient **1322.** The button is further operable to advance a plunger through the bolus reservoir **1312** to deliver a predetermined bolus volume to the patient **1322** through the injection flow path.

For one embodiment of an on-body injector for a basal injection, the injection device has an introducer port **1310** in fluid communication with a delivery tube **1320.** The on-body injector includes a pressurized reservoir **1306;** a flow restrictor **1308** disposed between the pressurized reservoir **1306** and the delivery tube **1320,** the flow restrictor **1308** being tubing having a length and interior diameter selected to provide a desired pressure drop; and a fill port **1304** in fluid communication with the pressurized reservoir.

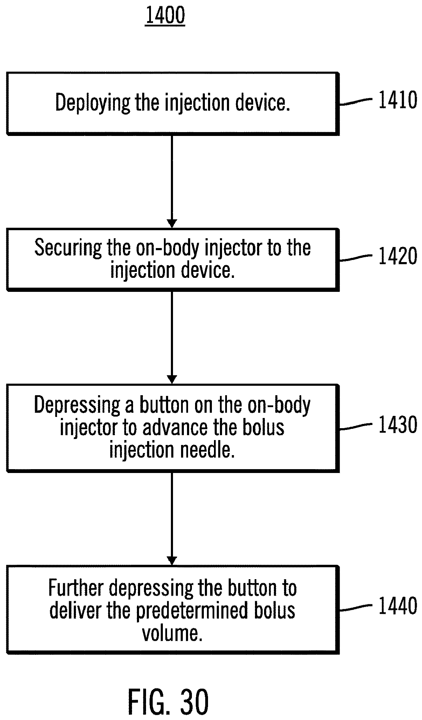

**FIG. 30** is a flow chart of a method of use for an on-body injector in accordance with the invention. The method **1400** is a method of use for an on-body injector with an injection device for delivering a predetermined bolus volume to a patient. The method **1400** includes deploying the injection device **1410** in the patient, the injection device having a delivery tube placed in the patient and an injection port in fluid communication with the delivery tube; securing the on-body injector to the injection device **1420,** the on-body injector having a bolus injection needle aligned with and spaced apart from the injection port; depressing a button on the on-body injector to advance the bolus injection needle **1430** into the injection port; and further depressing the button to deliver the predetermined bolus volume **1440** from the on-body injector through the bolus injection needle, through the delivery tube, and into the patient. The method **1400** can further include releasing the button to retract the bolus injection needle from the injection port.

The method **1400** can also include delivering a basal injection. In this embodiment, the injection device further includes an introducer port in fluid communication with the delivery tube, and the on-body injector further includes a pressurized reservoir in fluid communication with a basal injection needle inserted in the introducer port. The method **1400** further includes delivering a basal injection from the pressurized reservoir through the basal injection needle, through the delivery tube, and into the patient, the on-body injector can further include a fill port in fluid communication with the pressurized reservoir, in which case the method **1400** can further include delivering a therapeutic agent thorough the fill port into the pressurized reservoir.

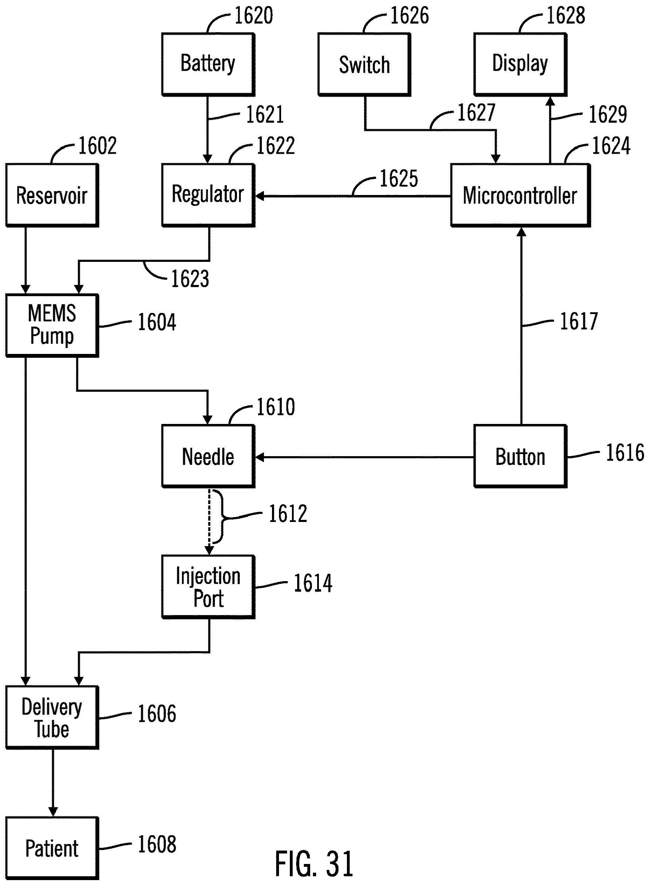

**FIGS. 31-38** illustrate an electronic injector for use with an injection device or for independent use. The electronic injector uses a Micro-Electro-Mechanical System (MEMS) pump to deliver a therapeutic agent from a reservoir to a patient.

**FIG. 31** is a block diagram of one embodiment of an injection device and electronic on-body injector made in accordance with the invention. **FIG. 31** illustrates the flow paths through and electrical signals for the injection device and electronic on-body injector, which can be used for a bolus injection and/or a basal injection. The injection device is deployed in the patient with a delivery tube placed subcutaneously and the electronic on-body injector is secured to the injection device.

The basal injection flow path includes a fluid reservoir **1602,** a MEMS pump **1604,** and a delivery tube **1606** supplying the patient **1608.** The bolus injection flow path includes the fluid reservoir **1602,** the MEMS pump **1604,** a bolus injection needle **1610,** a gap **1612,** an injection port **1614,** and the delivery tube **1606** supplying the patient **1608.** The bolus injection mechanism also includes a bolus needle button **1616** operable to advance the bolus injection needle **1610** and to activate the MEMS pump **1604** to deliver a predetermined bolus volume.

The electronic portion of the electronic on-body injector includes a battery **1620,** a regulator **1622,** and a microcontroller **1624.** The battery **1620** has a DC power output **1621** which is provided to the regulator **1622.** The battery **1620** is also operably connected (not shown) to provide power to the microcontroller **1624.** The regulator **1622** is operably connected to the battery **1620** to convert the DC power output **1621** to a pump drive signal **1623** in response to a regulator control signal **1625** from the microcontroller **1624.** The regulator **1622** provides the pump drive signal **1623** to the MEMS pump **1604.** The pump drive signal **1623** can be a basal pump drive signal or a bolus pump drive signal as required. The MEMS pump **1604** is responsive to the pump drive signal **1623** to control flow of the fluid from the fluid reservoir **1602,** through the MEMS pump **1604,** through the delivery tube **1606,** and into the patient **1608.**

The microcontroller **1624** controls the electronic on-body injector. The microcontroller **1624** receives a bolus needle button signal **1617** from the bolus needle button **1616,** which activates MEMS pump **1604** to deliver a predetermined bolus volume to the patient through the bolus injection flow path. The microcontroller **1624** provides the regulator control signal **1625** to the regulator **1622,** controlling the pump drive signal **1623.** The microcontroller **1624** can also optionally be responsive to a control switch signal **1627** from a control switch **1626** and/or can provide a display signal **1629** to a display **1628.** The control switch **1626** and the display **1628** can be used to provide input and output, respectively, to the electronic on-body injector. For example, the display **1628** can be used to display injection options, such as the basal injection rate, and control switch **1626** can be used to select one of the injection options. The microcontroller **1624** can also include or be associated with memory to store data and/or instructions. The microcontroller **1624** can also be operably connected to one or more sensors to monitor the patient **1608** and/or a wireless interface to communicate with one or more external sensors monitoring the patient **1608** or external communication and/or control systems, such as the internet, a continuous glucose monitoring system, a mobile device, or the like. Exemplary uses for a wireless interface providing communication between the electronic injector and an external system/device include calculating/setting dosages, tracking injection times and volumes, and/or sending reminders to a paired device (computer, phone, tablet, mobile device, or the like).

For basal injection, the fluid reservoir **1602** of the on-body injector provides the therapeutic agent to the patient **1608** through the basal injection flow path (the fluid reservoir **1602,** MEMS pump **1604,** and delivery tube **1606**). In one embodiment, the patient **1608** initiates the basal injection by pressing the control switch **1626,** which provides the control switch signal **1627** to the microcontroller **1624,** which provides the regulator control signal **1625** to the regulator **1622.** In this case, the regulator control signal **1625** is a basal regulator control signal and the regulator **1622** generates the pump drive signal **1623** as a basal pump drive signal. The MEMS pump **1604** delivers the desired basal injection to the patient **1608** in response to the basal pump drive signal. In another embodiment, the patient **1608** selects a desired basal flow rate using the control switch **1626** and the display **1628** before initiating the basal injection. Exemplary basal flow rates can include 20 units of insulin per 24 hours, 30 units of insulin per 24 hours, 40 units of insulin per 24 hours, or the like.

For bolus injection, the fluid reservoir **1602** of the on-body injector provides the therapeutic agent to the patient **1608** through the bolus injection flow path (the fluid reservoir **1602,** MEMS pump **1604,** bolus injection needle **1610,** gap **1612,** injection port **1614,** and delivery tube **1606**). The on-body injector delivers a predetermined bolus volume when the patient depresses the bolus needle button **1616** by activating the MEMS pump **1604** at a predetermined flow rate for a predetermined duration. When the bolus needle button **1616** is depressed, the tip of the bolus injection needle **1610** closes the gap **1612** between the bolus injection needle **1610** of the on-body injector and enters the injection port **1614** of the injection device to complete the bolus injection flow path. The gap **1612** as illustrated by the dashed lines between the bolus injection needle **1610** of the on-body injector and the injection port **1614** of the injection device is present when the bolus needle button **1616** is not depressed to prevent bolus injection unless the bolus needle button **1616** is depressed. Depressing the bolus needle button **1616** also delivers the bolus needle button signal **1617** to the microcontroller **1624,** which provides the regulator control signal **1625** to the regulator **1622.** In this case, the regulator control signal **1625** is a bolus regulator control signal and the regulator **1622** generates the pump drive signal **1623** as a bolus pump drive signal. The MEMS pump **1604** delivers the predetermined bolus volume to the patient **1608** in response to the bolus pump drive signal. In one embodiment, the bolus needle button **1616** triggers a switch in the path of button mechanism travel which starts the bolus injection when the bolus injection flow path is complete. In one example, the predetermined bolus volume is 2 units of insulin. In another embodiment, the patient **1608** selects a desired predetermined bolus volume using the control switch **1626** and the display **1628** before initiating the bolus injection. In this example, the basal and bolus drugs are the same drug, coming from the same fluid reservoir **1602** and going through the same flow path, but administered at different rates. The basal injection flows constantly at a very low rate. When a bolus injection is requested, the regulator **1622** changes the pump drive signal **1623** from the basal pump drive signal to a bolus delivery signal. When the bolus delivery is complete, the regulator **1622** changes the pump drive signal **1623** from the bolus pump drive signal to the basal delivery signal and basal injection resumes. In other embodiments, the basal injection flow path can include an orifice, check valve, or be sized so that the fluid does not flow forward or backward through the basal injection flow path during bolus injection.

The electronic on-body injector can optionally include a fill port (not shown) to fill or refill the fluid reservoir **1602.** In one embodiment, the fluid reservoir **1602** can be pre-filled with a therapeutic agent when the on-body injector is delivered to the patient, so that the fill port is used for refilling the fluid reservoir **1602.** In another embodiment, the fluid reservoir **1602** is empty when the electronic on-body injector is delivered to the patient, so that the fill port is used for initially filling the fluid reservoir **1602.** In yet another embodiment, the fill port is omitted and the electronic on-body injector is a single use device with the fluid reservoir **1602** pre-filled with a therapeutic agent.

For one embodiment of an electronic on-body injector for a bolus injection for use with a patient **1608** to deliver a fluid through an injection device, the injection device has an injection port **1614** in fluid communication with a delivery tube **1606** with the injection port **1614** lying on an injection axis. The electronic on-body injector includes a fluid reservoir **1602** operable to hold the fluid; a MEMS pump **1604** in fluid communication with the fluid reservoir **1602;** a bolus injection needle **1610** in fluid communication with the MEMS pump **1604,** the bolus injection needle **1610** having a bolus injection needle tip aligned with the injection port, the bolus injection needle **1610** being slideably biased away from the injection port to define a gap **1612** between the bolus injection needle tip and the injection port; and a bolus needle button **1616** operably connected to the bolus injection needle **1610** to slide the bolus injection needle **1610** along the injection axis. the bolus needle button **1616** is operable to advance the bolus injection needle tip to close the gap **1612** and advance the bolus injection needle tip into the injection port to form a bolus injection flow path from the fluid reservoir **1602,** through the MEMS pump **1604,** through the bolus injection needle **1610,** through the delivery tube **1606,** and into the patient **1608.** The bolus needle button **1616** is further operable to activate the MEMS pump **1604** to deliver a predetermined bolus volume to the patient **1608** through the bolus injection flow path in response to a bolus pump drive signal.

The electronic on-body injector for bolus injection can also include a battery **1620** having a DC power output **1621;** a regulator **1622** operably connected to the battery **1620** to convert the DC power output **1621** to the bolus pump drive signal in response to a regulator control signal **1625;** and a microcontroller **1624** operably connected to the regulator **1622** to provide the regulator control signal **1625.** The MEMS pump **1604** is responsive to the bolus pump drive signal to control flow of the fluid from the MEMS pump **1604** through the bolus injection flow path.

For one embodiment of an electronic on-body injector for a basal injection for use with a patient **1608** to deliver a fluid through an injection device, the injection device has a delivery tube **1606.** The electronic on-body injector includes a fluid reservoir **1602** operable to hold the fluid; and a MEMS pump **1604** in fluid communication with the fluid reservoir **1602** and the delivery tube **1606** to form a basal injection flow path from the fluid reservoir **1602,** through the MEMS pump **1604,** through the delivery tube **1606,** and into the patient **1608.** The MEMS pump **1604** is operable to deliver a basal injection to the patient **1608** through the basal injection flow path in response to a basal pump drive signal.

The electronic on-body injector for basal injection can also include a battery **1620** having a DC power output **1621;** a regulator **1622** operably connected to the battery **1620** to convert the DC power output **1621** to the basal pump drive signal in response to a regulator control signal **1625;** and a microcontroller **1624** operably connected to the regulator **1622** to provide the regulator control signal **1625.** The MEMS pump **1604** is responsive to the basal pump drive signal to control flow of the fluid from the MEMS pump **1604** through the basal injection flow path. Exemplary batteries **1620** include non-rechargeable alkaline batteries rechargeable lithium-ion batteries, rechargeable lithium ion polymer batteries, and the like. In one example, the battery capacity is in the range of 200-500 mAh, In one example, the battery dimensions are 15x40x5 mm. Those skilled in the art will appreciate that the battery type, capacity, and size can be selected as desired for a particular application. Exemplary microcontrollers **1624** include the CC2541 (Bluetooth/microprocessor combination) from Texas Instruments, PSoC®4 (microcontroller) from Cypress Semiconductor Corporation, or the like. Those skilled in the art will appreciate that the microcontrollers can be selected as desired for a particular application.

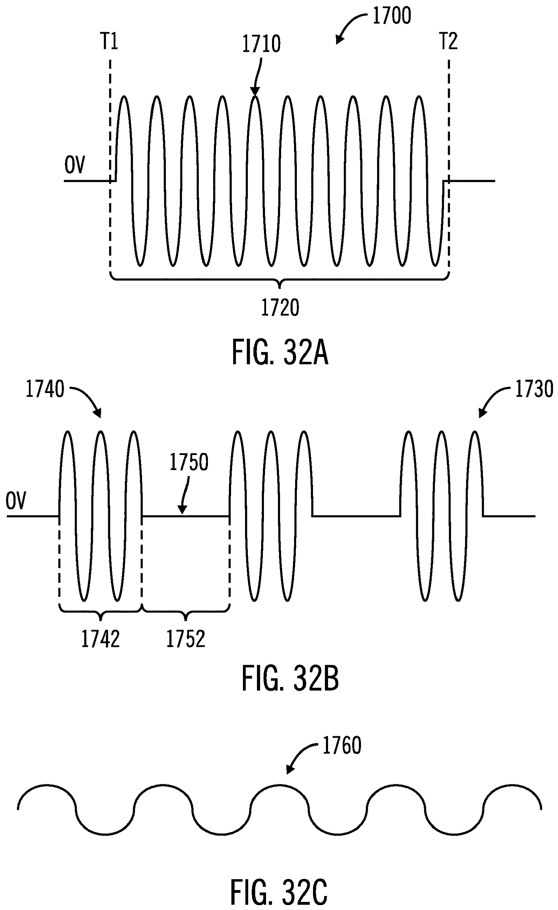

**FIGS. 32A-32C** are wave form diagrams of bolus, basal, and basal pump drive signals, respectively, for an electronic injector made in accordance with the invention. The frequency, amplitude, and duration of the AC portion of the pump drive signal determine the amount of fluid which is injected.

The bolus pump drive signal is selected to provide a predetermined bolus volume from the MEMS pump in response to a single bolus injection request from a patient. Referring to the example of **FIG. 32A****,** the bolus pump drive signal **1700** includes an AC power signal **1710** for a predetermined duration **1720** between time **T1** when the patient requests a bolus injection until the time T2 when the predetermined bolus volume has been delivered. The bolus pump drive signal **1700** has a value of 0 V DC before and after the AC power signal **1710.** The MEMS pump moves the fluid when the AC power signal **1710** is active. In this example, the AC power signal **1710** is a sine wave. Those skilled in the art will appreciate that the pump drive signal can have any AC waveform as desired for a particular application. For example, the AC waveform can be a sine wave, a saw tooth wave, a square wave, or the like.

The basal pump drive signal is selected to provide a desired basal flow rate from the MEMS pump. Referring to the example of **FIG. 32B****,** the basal pump drive signal **1730** includes a series of AC power signals **1740** of AC power duration **1742** alternating with zero volt power signals **1750** of zero volt power duration **1752.** The time of AC power duration **1742** and/or 0 V power duration **1752** can be selected to provide the desired basal flow rate. The MEMS pump moves the fluid when each of the AC power signals **1740** is active. In this example, the AC power signal **1740** is a sine wave. Those skilled in the art will appreciate that the pump drive signal can have any AC waveform as desired for a particular application. For example, the AC waveform can be a sine wave, a saw tooth wave, a square wave, or the like.

**FIG. 32C** is an example of a continuous basal pump drive signal, which provides a continuous AC waveform. The basal pump drive signal **1760** has a frequency and/or amplitude which is substantially less than the AC power signal of **FIG. 32A** or **FIG. 32B****,** so that the basal pump drive signal **1760** is continuously applied to the MEMS pump to provide a desired basal flow rate from the MEMS pump. In this example of **FIG. 32C****,** the basal pump drive signal **1760** is a sine wave. Those skilled in the art will appreciate that the basal pump drive signal can have any AC waveform as desired for a particular application. For example, the basal pump drive signal can be a sine wave, a saw tooth wave, a square wave, or the like.

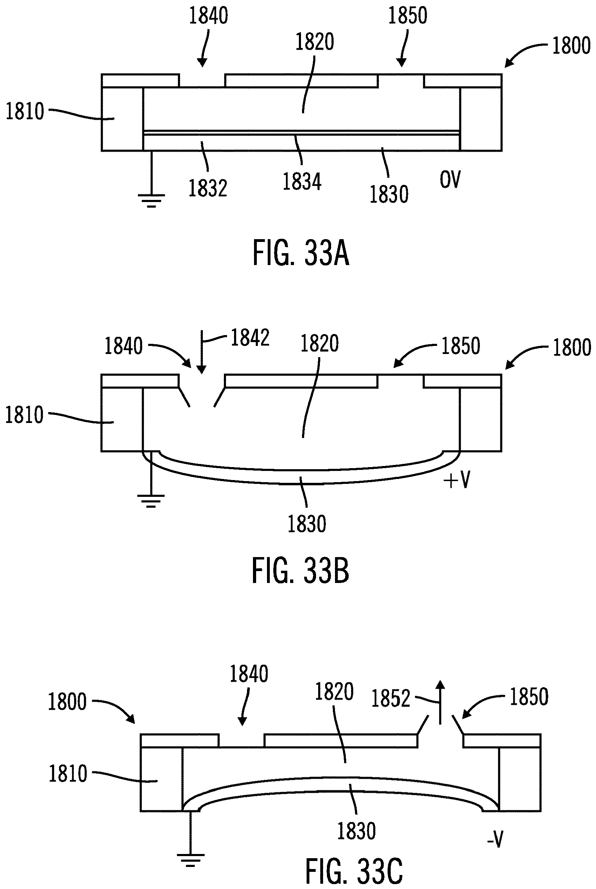

**FIGS. 33A-33C****,** in which like elements share like reference numbers, are schematic diagrams of a piezoelectric MEMS pump for use in an electronic injector made in accordance with the invention. **FIG. 33A** illustrates the MEMS pump at rest, **FIG. 33B** illustrates the MEMS pump during fluid intake, and **FIG. 33C** illustrates the MEMS pump during fluid output.

Referring to **FIG. 33A** illustrating the MEMS pump at rest, the piezoelectric MEMS pump **1800** includes a case **1810** defining a working chamber **1820,** the working chamber **1820** having a piezoelectric wall **1830** responsive to a first pump drive signal voltage and a second pump drive signal voltage; a one way inlet valve **1840** operable to permit flow of the fluid into the working chamber **1820** and to block reverse flow of the fluid from the working chamber **1820;** and a one way outlet valve **1850** operable to permit flow of the fluid from the working chamber **1820** and to block reverse flow of the fluid into the working chamber **1820.** The one way inlet valve **1840** is in fluid communication with the fluid reservoir (not shown). When the piezoelectric MEMS pump **1800** is used for bolus injection, the one way outlet valve **1850** is in fluid communication with the bolus injection needle (not shown). When the piezoelectric MEMS pump **1800** is used for basal injection, the one way outlet valve **1850** is in fluid communication with the delivery tube (not shown). In one embodiment, the piezoelectric wall **1830** includes a piezoelectric disk **1832** with a membrane **1834** sealing the working chamber **1820.** The working chamber **1820** can be etched directly out of the silicon base material. The flexible membrane **1834** can be a thin layer of silicon or silicon dioxide. The piezoelectric disk **1832** can be attached to the flexible membrane **1834.**

Referring to **FIG. 33B** illustrating the MEMS pump during fluid intake, the piezoelectric wall **1830** flexes outward to increase volume of the working chamber **1820** in response to the first pump drive signal voltage (+V to ground across the piezoelectric wall **1830)** to draw the fluid through the one way inlet valve to the working chamber **1820** as indicated by the arrow **1842.**

Referring to **FIG. 33C** illustrating the MEMS pump during fluid output, the piezoelectric wall **1830** flexes inward to decrease the volume of the working chamber **1820** in response to the second pump drive signal voltage (-V to ground across the piezoelectric wall **1830**) to force the fluid from the working chamber **1820** through the one way outlet valve **1850** as indicated by the arrow **1852.**

Alternately applying the first pump drive signal voltage and the second pump drive signal voltage causes the piezoelectric wall **1830** to oscillate, pumping fluid through the piezoelectric MEMS pump **1800.** In one embodiment, the first pump drive signal voltage and the second pump drive signal voltage are applied as a pump drive signal as a square wave. The piezoelectric MEMS pump **1800** can be fabricated using standard semiconductor techniques, resulting in a pump of reduced size and improved accuracy compared to standard mechanical pumps. In one example, the piezoelectric MEMS pump **1800** can deliver flow rates up to 3 milliliters per minute, which is the equivalent of 300 units of insulin per minute or 5 units of insulin per second. Those skilled in the art will appreciate that the MEMS pump is not limited to a piezoelectric MEMS pump, but can be a bimetallic pump, an electrostatic pump, a thermopneumatic pump, an electromagnetic pump, a phase change pump, or the like, as desired for a particular application.

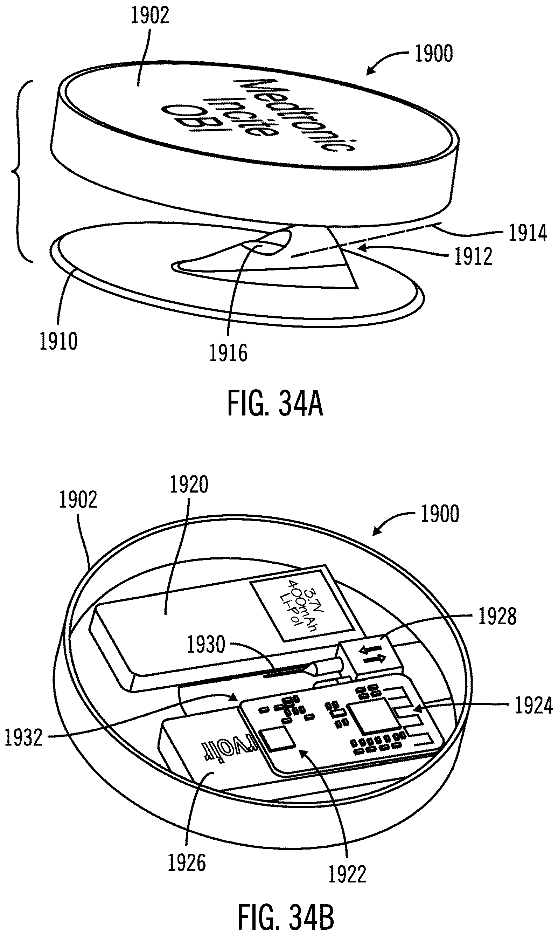

**FIGS. 34A-34C****,** in which like elements share like reference numbers, are an exploded perspective view, a partial perspective view, and a partial perspective view of an injection device and electronic on-body injector made in accordance with the invention. In this embodiment, the electronic on-body injector is the electronic injector.

Referring to **FIG. 34A****,** the electronic on-body injector **1900** is illustrated separated from the injection device **1910.** The electronic on-body injector **1900** has a housing **1902** to contain the internal components of the electronic on-body injector **1900.** The injection device **1910** has an injection port **1912** lying on an injection axis **1914** as illustrated by the dashed line. The injection device **1910** in this example also has an introducer port **1916.**

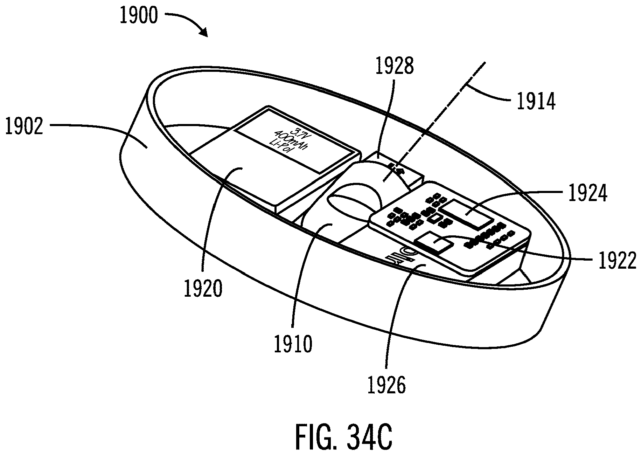

Referring to **FIG. 34B****,** which illustrates the electronic on-body injector **1900** with the top of the housing **1902** removed, the interior of the housing **1902** of the electronic on-body injector **1900** encloses the battery **1920,** the regulator **1922,** the microcontroller **1924,** the fluid reservoir **1926,** and the MEMS pump **1928.** The bolus injection needle **1930** is partially enclosed within the housing **1902** with the bolus injection needle tip **1932** extending from the housing **1902** to access the injection device. Various controls and indicators (not shown), such as the bolus needle button, control switches, displays, and the like, can extend through and/or be positioned upon the housing **1902.** **FIG. 34C** illustrates the electronic on-body injector **1900** in place on the injection device **1910.** The bolus injection needle (not shown) is aligned with the injection axis **1914** of the injection device **1910.**

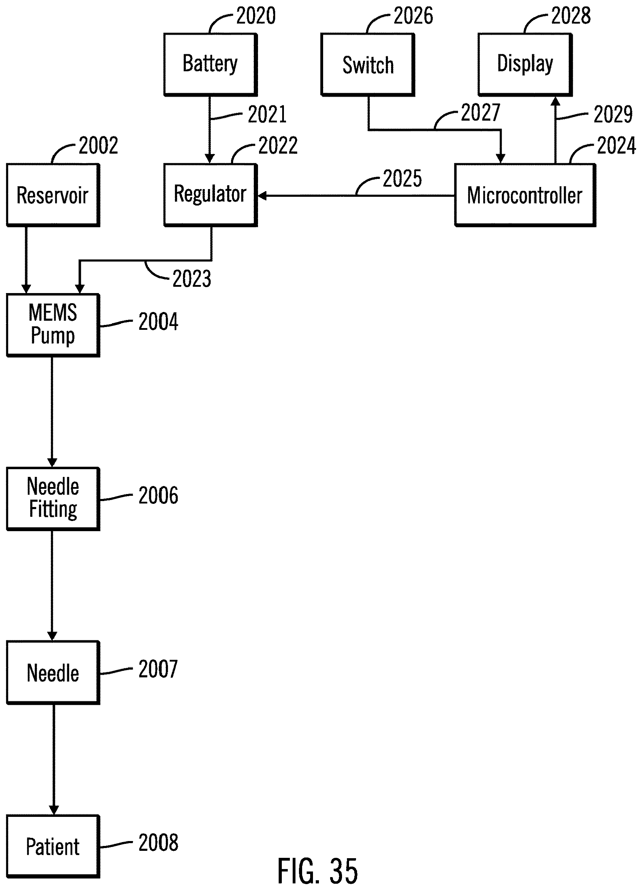

**FIG. 35** is a block diagram of one embodiment of an electronic injector made in accordance with the invention. **FIG. 35** illustrates the flow paths through and electrical signals for the electronic injector, which can be used for injection of a therapeutic agent.

The basal injection flow path includes a fluid reservoir **2002,** a MEMS pump **2004,** a needle fitting **2006,** and an injection needle **2007** supplying the patient **2008.** In one embodiment, the injection needle **2007** is detachable from the needle fitting **2006** so that the injection needle **2007** can be replaced at a desired frequency, e.g., after each injection. In another embodiment, the injection needle **2007** is permanently attached to the needle fitting **2006.** In yet another embodiment, no injection needle is used, but the tip of the electronic injector is adapted for use as a needleless pen injector as described in conjunction with **FIGS. 11** **&** **12** above.

Referring to **FIG. 35****,** the electronic portion of the electronic injector includes a battery **2020,** a regulator **2022,** and a microcontroller **2024.** The battery **2020** has a DC power output **2021** which is provided to the regulator **2022.** The battery **2020** is also operably connected (not shown) to provide power to the microcontroller **2024.** The regulator **2022** is operably connected to the battery **2020** to convert the DC power output **2021** to a pump drive signal **2023** in response to a regulator control signal **2025** from the microcontroller **2024.** The regulator **2022** provides the pump drive signal **2023** to the MEMS pump **2004.** The MEMS pump **2004** is responsive to the pump drive signal **2023** to control flow of the fluid from the fluid reservoir **2002,** through the MEMS pump **2004,** through the needle fitting **2006,** through the needle **2007,** and into the patient **2008.** In one embodiment, the MEMS pump **2004** is a piezoelectric MEMS pump as described in conjunction with **FIGS. 33A-33C** above. In other embodiments, the MEMS pump **2004** can be a bimetallic pump, an electrostatic pump, a thermopneumatic pump, an electromagnetic pump, a phase change pump, or the like, as desired for a particular application.

Referring to **FIG. 35****,** the microcontroller **2024** controls the electronic injector. The microcontroller **2024** provides the regulator control signal **2025** to the regulator **2022,** controlling the pump drive signal **2023.** The microcontroller **2024** can be responsive to a control switch signal **2027** from a control switch **2026** and/or can provide a display signal **2029** to a display **2028.** The control switch **2026** and the display **2028** can be used to provide input and output, respectively, to the electronic injector. For example, the display **2028** can be used to display injection options, such as the bolus injection volume, and control switch **2026** can be used to select one of the injection options. The microcontroller **2024** can also include or be associated with memory to store data and/or instructions. The microcontroller **2024** can also be operably connected to one or more sensors to monitor the patient **2008** and/or a wireless interface to communicate with one or more external sensors monitoring the patient **2008** or external communication and/or control systems, such as the internet, a continuous glucose monitoring system, a mobile device, or the like. Exemplary uses for a wireless interface providing communication between the electronic injector and an external system/device include calculating/setting dosages, tracking injection times and volumes, and/or sending reminders to a paired device (computer, phone, tablet, mobile device, or the like).

For bolus injection, the fluid reservoir **2002** of the on-body injector provides the therapeutic agent to the patient **2008** through the injection flow path (the fluid reservoir **2002,** MEMS pump **2004,** needle fitting **2006,** and injection needle **2007**). In one embodiment, the patient **2008** initiates a bolus injection by pressing the control switch **2026,** which provides the control switch signal **2027** to the microcontroller **2024,** which provides the regulator control signal **2025** to the regulator **2022.** In this case, the regulator control signal **2025** is a bolus regulator control signal and the regulator **2022** generates the pump drive signal **2023** as a bolus pump drive signal. The MEMS pump **2004** delivers the desired bolus injection to the patient **2008** in response to the bolus pump drive signal. In another embodiment, the patient **2008** selects a predetermined bolus volume using the control switch **2026** and the display **2028** before initiating the bolus injection. In one example, the predetermined bolus volume is 2 units of insulin.

The electronic injector can optionally include a fill port (not shown) to fill or refill the fluid reservoir **2002.** In one embodiment, the fluid reservoir **2002** can be pre-filled with a therapeutic agent when the on-body injector is delivered to the patient, so that the fill port is used for refilling the fluid reservoir **2002.** In another embodiment, the fluid reservoir **2002** is empty when the electronic injector is delivered to the patient, so that the fill port is used for initially filling the fluid reservoir **2002.** In yet another embodiment, the fill port is omitted and the electronic injector is a single use device with the fluid reservoir **2002** pre-filled with a therapeutic agent.

For one embodiment of an electronic injector for use with a patient **2008** to deliver a fluid, the electronic injector includes a fluid reservoir **2002** operable to hold the fluid; a MEMS pump **2004** in fluid communication with the fluid reservoir **2002;** a needle fitting **2006** adapted to receive an injection needle, the needle fitting **2006** being in fluid communication with the MEMS pump **2004;** a battery **2020** having a DC power output **2021;** a regulator **2022** operably connected to the battery **2020** to convert the DC power output **2021** to a pump drive signal **2023** in response to a regulator control signal **2025;** a microcontroller **2024** operably connected to the regulator **2022** to provide the regulator control signal **2025;** and a housing to enclose the battery **2020,** the regulator **2022,** the microcontroller **2024,** the fluid reservoir **2002,** and the MEMS pump **2004.** The MEMS pump **2004** is responsive to the pump drive signal **2023** to control flow of the fluid from the fluid reservoir **2002,** through the MEMS pump **2004,** through the injection needle **2007,** and into the patient **2008.**

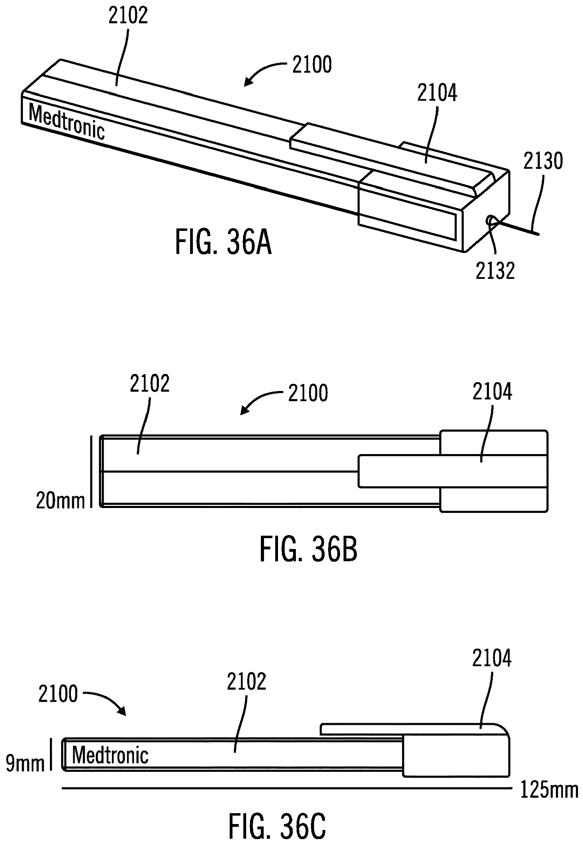

**FIGS. 36A-36D****,** in which like elements share like reference numbers, are a perspective view, a top view, a side view, and a partial perspective view of an electronic injector made in accordance with the invention. In this example, the electronic injector has a pen form factor, which in this example has a length less than or equal to 125 mm, a width less than or equal to 20 mm, and a height less than or equal to 9 mm.

Referring to **FIGS. 36A-36C****,** the electronic injector **2100** has a housing **2102** to contain the internal components of the electronic injector **2100.** The injection needle **2130** is attached to the electronic injector **2100** at the needle fitting **2132.** In this example, the electronic injector **2100** also includes a cap **2104** removeable from the housing **2102.** In one embodiment, the housing **2102**can include a control switch (not shown) which can be used to initiate an injection.

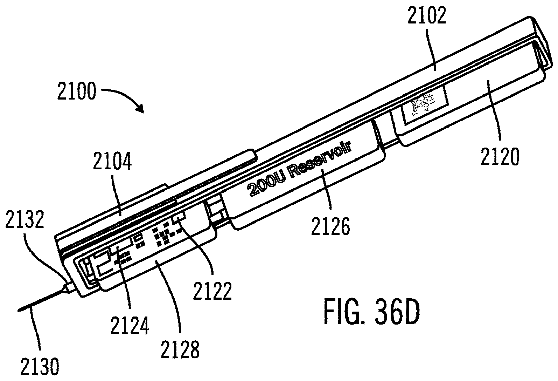

Referring to **FIG. 36D****,** which illustrates the electronic injector **2100** with a portion of the housing **2102** removed, the interior of the housing **2102** of the electronic injector **2100** encloses the battery **2120,** the regulator **2122,** the microcontroller **2124,** the fluid reservoir **2126,** and the MEMS pump **2128.** Various controls and indicators (not shown), such as control switches, displays, and the like, can extend through and/or be positioned upon the housing **2102.**

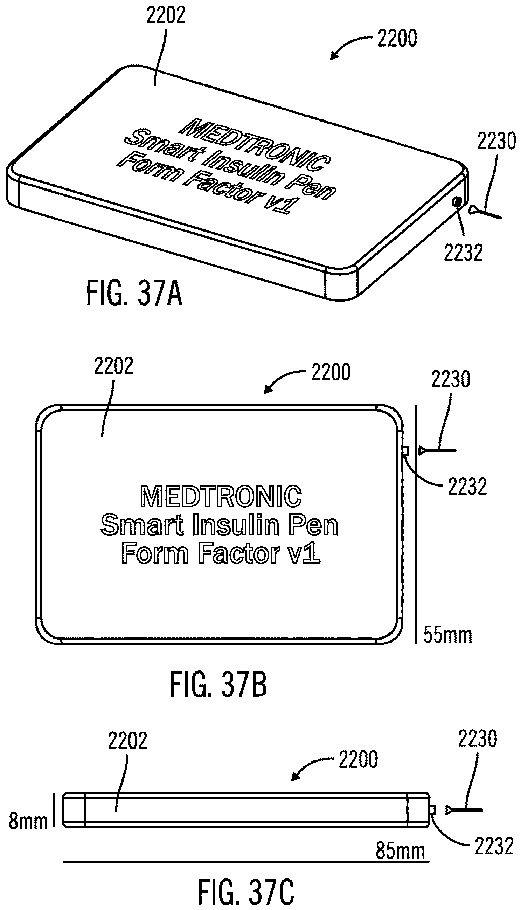

**FIGS. 37A-37E****,** in which like elements share like reference numbers, are a perspective view, a top view, a side view, an exploded perspective view, and a partial top view of an electronic injector made in accordance with the invention. In this example, the electronic injector has a card form factor, which in this example has a length less than or equal to 85 mm, a width less than or equal to 55 mm, and a height less than or equal to 8 mm.

Referring to **FIGS. 37A-37C****,** the electronic injector **2200** has a housing **2202** to contain the internal components of the electronic injector **2200.** The injection needle **2230** can be attached to the electronic injector **2200** at the needle fitting **2232.**

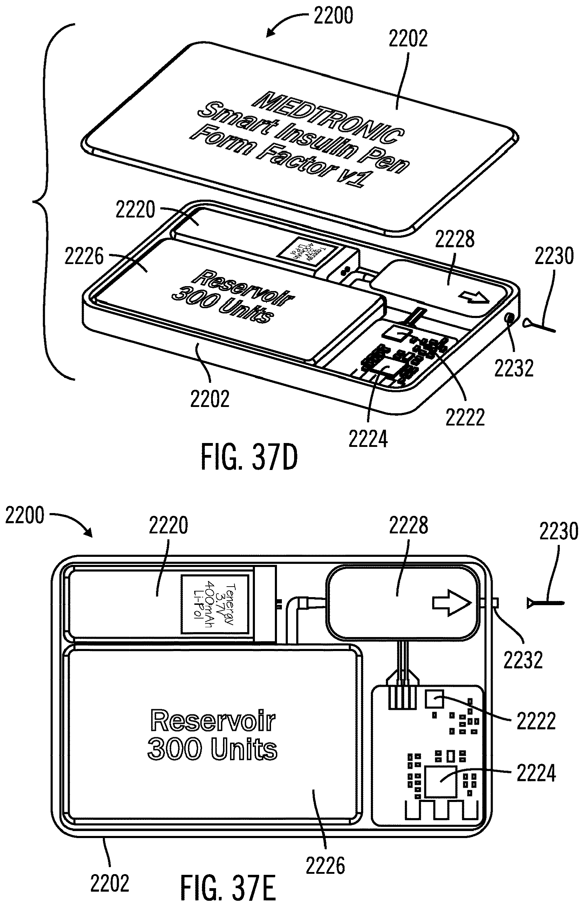

Referring to **FIGS. 37D & 37E****,** which illustrate the electronic injector **2200** with a top portion of the housing **2202** removed, the interior of the housing **2202** of the electronic injector **2200** encloses the battery **2220,** the regulator **2222,** the microcontroller **2224,** the fluid reservoir **2226,** and the MEMS pump **2228.** Various controls and indicators (not shown), such as control switches, displays, and the like, can extend through and/or be positioned upon the housing **2202.**

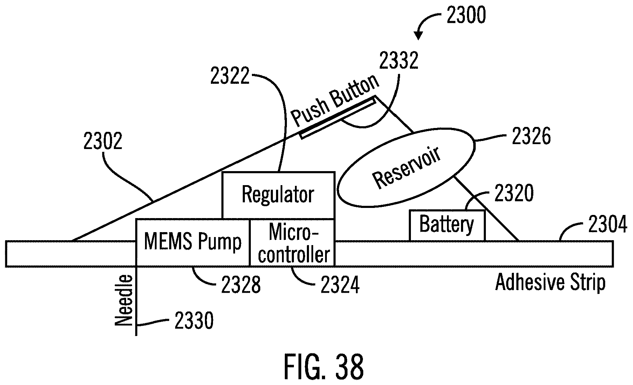

**FIG. 38** is a schematic cross section view of an electronic injector made in accordance with the invention. The electronic injector includes an adhesive patch operable to secure the housing to the patient.

The electronic injector **2300** has a housing **2302** to contain the internal components of the electronic injector **2300.** The injection needle **2330** protrudes through the adhesive strip **2304,** which forms the bottom of the housing **2302** for adhesively attaching the electronic injector **2300** to the patient. The interior of the housing **2302** of the electronic injector **2300** encloses the battery **2320,** the regulator **2322,** the microcontroller **2324,** the fluid reservoir **2326,** and the MEMS pump **2328.** Various controls and indicators

(not shown), such as control switches, displays, and the like, can extend through and/or be positioned upon the housing **2302.** In one embodiment, the electronic injector **2300** includes a push button **2332** which can be used to initiate delivery of a therapeutic agent to the patient.

The electronic injector as described in conjunction with **FIGS. 31-38** above can be extended to include additional features for convenience, ease-of-use, and safety.

The electronic injector can include wireless functionality, allowing communication with local or remote communication devices. Examples of wireless connections include Bluetooth, Bluetooth Low Energy, Near Field Communication or 802.11 Wi-Fi, and the like. This wireless communication can be used to pull injection data from the electronic injector and to interface with the electronic injector, including inputting data to calculate injection dosages, directly inputting injection dosages, setting timers, reminders, and safety lockouts. In one embodiment, the electronic injector can be paired with an on-body continuous glucose monitor to calculate the desired bolus injection volume for the electronic injector and avoid manual entry. In this embodiment, both the Continuous Glucose Monitor (CGM) and the injector can be paired with a wireless device running a control application. The wireless device can read in the current glucose level of the patient from the CGM and the patient can manually enter their weight/insulin resistance information (which can be saved for future use and updated as needed) along with their intended sugar intake, the mobile device control application can then calculate the injection volume and send that information back to the electronic injector. In this embodiment, neither the electronic injector nor the CGM has the computational capability to do this calculation. In another embodiment, the CGM can connect directly to the electronic injector. The patient can perform an initial step of loading in their weight and treatment resistance information. During normal use, the patient would only enter the amount of food they plan to consume. The electronic injector can then calculate the correct dosage for the patient. In another embodiment, the electronic injector can wirelessly connect to the CGM to read the patient's current glucose levels, then can take direct patient input on incoming sugars. The electronic injector can then perform the dosage calculation with no other wireless device involved. In another embodiment, the electronic injector can be paired with a communication device (computer, phone, tablet, etc.) which tracks usage data obtained by the electronic injector. Exemplary data include tracking of injection times and injection amounts, which can be used to establish trends and/or to verify that proper injections occurred at proper times. Such tracking could be particularl useful in caring for the young and the elderly. In yet another embodiment, the electronic injector can obtain and/or provide data to remote medical databases, such as the Medtronic CareLink Network, to provide an easy and quick interface between the patient and physician in an effort to continuously manage the patient's treatment. This would be particularly useful for new patients that need to make frequent adjustments to their dosage until the patient and doctor determine the correct dosages to use.

The electronic injector can also include safety features. In one embodiment, the electronic injector can remind the patient when it is the proper time for a basal injection by use of a vibration, auditory, and/or visual signal at a preset or predetermined time. The electronic injector can also verify that injections occur at the proper injection time with the proper injection amount, and provide the data to remote medical databases or control applications over a paired communication device. In another embodiment, the electronic injector can provide a warning or injection lockout when a second injection is attempted within a certain time period, preventing accidental double injections. In yet another embodiment when the therapeutic agent is provided in a replaceable reservoir, the electronic injector can detect the type of replaceable reservoir and/or recognize the type of insulin or other therapeutic agent being used, and adjust the operation of the electronic injector automatically. Each reservoir can have a unique ID tag, which can take the form of an RFID tag, EEPROM, a variable resistance, a small optical tag, or the like, that the electronic injector can scan to identify the specific reservoir type, contents, and/or expiration date.

The electronic injector can also include device monitoring features. In one embodiment, the electronic injector can verify the flow rate of the therapeutic agent using a MEMS flow meter to assure that it is correct. When the flow rate is too high, the electronic injector can stop the injection to avoid an excessive delivery of the therapeutic agent. When the flow rate is too low, the electronic injector can stop the pump to avoid build up of excessive delivery pressure due to an obstruction in the flowpath, such as a kink in the tubing. In either situation, the electronic injector can send a warning to the patient via a vibration, auditory signal, and/or visual signal through either the electronic injector or paired mobile device indicating that the injection has stopped. In another embodiment, the electronic injector can have a temperature sensor that monitors the temperature of the therapeutic agent within the reservoir. When the therapeutic agent temperature rises above or falls below recommended thresholds, the electronic injector can warn the patient via a vibration, auditory signal, and/or visual signal that the therapeutic agent may no longer be safe to use. In another embodiment, the electronic injector can provide a low battery warning to warn the patient that the electronic injector may not be available for service. This warning can be a vibration, auditory signal, and/or visual signal. This warning can also pop up as a warning, such as a vibration, auditory signal, and/or visual signal, on a wirelessly connected mobile device. In yet another embodiment, the electronic injector can check the identity and/or the expiration date of the therapeutic agent when the therapeutic agent is provided in a replaceable reservoir. This information can be included in the unique ID tag described above.

The electronic injector can also include green technology features. In one embodiment, the electronic injector can harvest energy to recharge or replace the battery. The electronic injector can turn mechanical motion (button press, body motions) into electrical energy through a piezo energy harvesting module. In another embodiment, the electronic injector can use rechargeable batteries that can be recharged via an AC wall adapter, or a USB, mini USB or micro USB connector.

The electronic injector can also include therapeutic agent flexibility features. The electronic injector can work with existing therapeutic agents and new therapeutic agent coming onto the market. Along with insulin for diabetes treatment, the electronic injector can work with glucagon-like peptide-1 (GLP-1) or Amylin pancreatic hormone. The electronic injector can also be used with other therapeutic agents such as injectable pain medication, steroids, Botox, or the like. In one embodiment, the electronic injector can adapt operation to provide a maximum flow rate and/or lockout timer through internal control valves and utilizing a clock timer within the electronic injector to prevent abuse of the therapeutic agent. In one embodiment, the electronic injector can include dual reservoirs so that one electronic injector can provide different therapeutic agents to a patient requiring more than one type of therapeutic agent. In one example, one reservoir can house fast acting bolus insulin, e.g., Novolog, and the other reservoir can house slow acting basal insulin, e.g., Lantix.

The electronic injector can include convenience features to encourage the patient to use the device. In one embodiment, the electronic injector would be disposable with all the parts being prepackaged (including the therapeutic agent in the reservoir) and would be tossed away as soon as the therapeutic agent is used up or expires. In another embodiment, the electronic injector would be durable with a replaceable cartridge containing just the reservoir and MEMS micropump, which would snap into place and would require only an electrical connection to the durable parts of the pump. In this embodiment, the entire flow path (reservoir, MEMS pump, and injection needle port) for the therapeutic agent can be contained within the disposable cartridge, so that only an external electrical signal from the durable portion of the electronic injector is required to control the pump. In yet another embodiment, the electronic injector would be durable and only a replaceable cartridge including the therapeutic agent would be replaced. In this embodiment, the cartridge can make a mechanical connection to create the fluid flow path from the reservoir to the MEMS pump.

It is important to note that **FIGS. 1-38** illustrate specific applications and embodiments of the invention, and are not intended to limit the scope of the present disclosure or claims to that which is presented therein. Upon reading the specification and reviewing the drawings hereof, it will become immediately obvious to those skilled in the art that myriad other embodiments of the invention are possible, and that such embodiments are contemplated and fall within the scope of the presently claimed invention.

While the embodiments of the invention disclosed herein are presently considered to be preferred, various changes and modifications can be made without departing from the spirit and scope of the invention. The scope of the invention is indicated in the appended claims, and all changes that come within the meaning and range of equivalents are intended to be embraced therein.

The paragraphs that follow define further embodiments forming part of the present disclosure.
Paragraph 1. An on-body injector for use with a patient with an injection device having an injection port in fluid communication with a delivery tube, the injection port lying on an injection axis, the on-body injector comprising:
   a bolus reservoir;
   a bolus injection needle in fluid communication with the bolus reservoir, the bolus injection needle having a bolus injection needle tip aligned with the injection port, the bolus injection needle being slideably biased away from the injection port to define a gap between the bolus injection needle tip and the injection port; and
   a button operably connected to the bolus injection needle to slide the bolus injection needle along the injection axis;
   wherein the button is operable to advance the bolus injection needle tip to close the gap and advance the bolus injection needle tip into the injection port to form an injection flow path from the bolus reservoir, through the bolus injection needle, through the delivery tube, and into the patient; and
   wherein the button is further operable to advance a plunger through the bolus reservoir to deliver a predetermined bolus volume to the patient through the injection flow path.
Paragraph 2. The on-body injector of paragraph 1 wherein the plunger blocks the injection flow path when the plunger is at a final position in the bolus reservoir when the predetermined bolus volume has been delivered.
Paragraph 3. The on-body injector of paragraph 1 wherein the predetermined bolus volume is 2 units of insulin.
Paragraph 4. The on-body injector of paragraph 1 further comprising a pressurized reservoir in fluid communication with the bolus reservoir.
Paragraph 5. The on-body injector of paragraph 4 wherein the injection device has an introducer port in fluid communication with the delivery tube, the pressurized reservoir being in fluid communication with the delivery tube through a flow restrictor.
Paragraph 6. The on-body injector of paragraph 5 wherein the flow restrictor is tubing having a length and interior diameter selected to provide a desired pressure drop.
Paragraph 7. The on-body injector of paragraph 5 wherein the flow restrictor is selected to provide a basal flow rate selected from the group consisting of 20 units of insulin per 24 hours, 30 units of insulin per 24 hours, and 40 units of insulin per 24 hours.
Paragraph 8. The on-body injector of paragraph 4 further comprising a fill port in fluid communication with the pressurized reservoir.
Paragraph 9. The on-body injector of paragraph 4 further comprising a housing defining an interior track, wherein the pressurized reservoir is a spring coil operable to at least partially uncoil within the interior track when the pressurized reservoir is at least partially filled with a therapeutic agent.
Paragraph 10. The on-body injector of paragraph 1 further comprising a spring operable to slideably bias the bolus injection needle away from the injection port.
Paragraph 11. The on-body injector of paragraph 1 further comprising a lock operable to secure the on-body injector to the injection device.
Paragraph 12. An on-body injector for use with a patient with an injection device having an introducer port in fluid communication with a delivery tube, the on-body injector comprising:
   a pressurized reservoir;
   a flow restrictor disposed between the pressurized reservoir and the delivery tube, the flow restrictor being tubing having a length and interior diameter selected to provide a desired pressure drop; and
   a fill port in fluid communication with the pressurized reservoir.
Paragraph 13. The on-body injector of paragraph 12 further comprising a housing defining an interior track, wherein the pressurized reservoir is a spring coil operable to at least partially uncoil within the interior track when the pressurized reservoir is at least partially filled with a therapeutic agent.
Paragraph 14. The on-body injector of paragraph 12 wherein the injection device further has an injection port in fluid communication with the delivery tube, the injection port lying on an injection axis, the on-body injector further comprising:
   a bolus reservoir in fluid communication with the pressurized reservoir;
   a bolus injection needle in fluid communication with the bolus reservoir, the bolus injection needle having a bolus injection needle tip aligned with the injection port, the bolus injection needle being slideably biased away from the injection port to define a gap between the bolus injection needle tip and the injection port; and
   a button operably connected to the bolus injection needle to slide the bolus injection needle along the injection axis;
   wherein the button is operable to advance the bolus injection needle tip to close the gap and advance the bolus injection needle tip into the injection port to form an injection flow path from the bolus reservoir, through the bolus injection needle, through the delivery tube, and into the patient; and
   wherein the button is further operable to advance a plunger through the bolus reservoir to deliver a predetermined bolus volume to the patient through the injection flow path.
Paragraph 15. The on-body injector of paragraph 14 wherein the plunger blocks the injection flow path when the plunger is at a final position in the bolus reservoir when the predetermined bolus volume has been delivered.
Paragraph 16. The on-body injector of paragraph 12 further comprising a lock operable to secure the on-body injector to the injection device.
Paragraph 17. A method of use for an on-body injector with an injection device for delivering a predetermined bolus volume to a patient, the method comprising:
   deploying the injection device in the patient, the injection device having a delivery tube placed in the patient and an injection port in fluid communication with the delivery tube;
   securing the on-body injector to the injection device, the on-body injector having a bolus injection needle aligned with and spaced apart from the injection port; depressing a button on the on-body injector to advance the bolus injection needle into the injection port; and
   further depressing the button to deliver the predetermined bolus volume from the on-body injector through the bolus injection needle, through the delivery tube, and into the patient.
Paragraph 18. The method of paragraph 17 further comprising releasing the button to retract the bolus injection needle from the injection port.
Paragraph 19. The method of paragraph 17 wherein the injection device further comprises an introducer port in fluid communication with the delivery tube, and the on- body injector further comprises a pressurized reservoir in fluid communication with a basal injection needle inserted in the introducer port, the method further comprising delivering a basal injection from the pressurized reservoir through the basal injection needle, through the delivery tube, and into the patient.
Paragraph 20. The method of paragraph 19 wherein the on-body injector further comprises a fill port in fluid communication with the pressurized reservoir, the method further comprising delivering a therapeutic agent thorough the fill port into the pressurized reservoir.

## Claims

1. An on-body injector (192) for use with a patient with an injection device having an injection port in fluid communication with a delivery tube (150), the injection port (140) lying on an injection axis (144), the on-body injector (192) comprising:
a bolus reservoir (1100);
a bolus injection needle (1040) in fluid communication with the bolus reservoir (1100), the bolus injection needle (1040) having a bolus injection needle tip (1042) aligned with the injection port, the bolus injection needle being slideably biased away from the injection port (140) to define a gap (1120) between the bolus injection needle tip (1042) and the injection port (140); and
a button (1030) operably connected to the bolus injection needle (1040) to slide the bolus injection needle (1040) along the injection axis (144);
wherein the button (1030) is operable to advance the bolus injection needle tip (1042) to close the gap (1120) and advance the bolus injection needle tip into the injection port to form an injection flow path from the bolus reservoir (1100), through the bolus injection needle (1040), through the delivery tube (150), and into the patient;
wherein the button (1030) is further operable to advance a plunger (1110) through the bolus reservoir (1100) to deliver a predetermined bolus volume to the patient through the injection flow path; and
a pressurized reservoir (1070) in fluid communication with the bolus reservoir.

2. The on-body injector of claim 1 wherein the injection device has an introducer port in fluid communication with the delivery tube (150), the pressurized reservoir (1070) being in fluid communication with the delivery tube through a flow restrictor (1060).

3. The on-body injector of claim 2 wherein the flow restrictor (1060) is tubing having a length and interior diameter selected to provide a desired pressure drop.

4. The on-body injector of claim 2 wherein the flow restrictor (1060) is selected to provide a basal flow rate selected from the group consisting of 20 units of insulin per 24 hours, 30 units of insulin per 24 hours, and 40 units of insulin per 24 hours.

5. The on-body injector of claim 1 further comprising a fill port (1050) in fluid communication with the pressurized reservoir (1070).

6. The on-body injector of claim 1 further comprising a housing defining an interior track (1080), wherein the pressurized reservoir (1070) is a spring coil operable to at least partially uncoil within the interior track when the pressurized reservoir is at least partially filled with a therapeutic agent.

7. The on-body injector of claim 1 wherein the injection device has an introducer port in fluid communication with the delivery tube (150), the pressurized reservoir (1070) being in fluid communication with the delivery tube through a flow restrictor (1060), the flow restrictor (1060) comprising tubing having a length and interior diameter selected to provide a desired pressure drop; and
further comprising a fill port (1050) in fluid communication with the pressurized reservoir (1070).

8. The on-body injector of claim 7 wherein the plunger (1110) blocks the injection flow path when the plunger is at a final position in the bolus reservoir (1100) when the predetermined bolus volume has been delivered.
